# EUROPÄISCHE PATENTANMELDUNG

(11) **EP 2 428 118 A2**
(43) Veröffentlichungstag der Anmeldung: **14.03.2012**
(21) Anmeldenummer: 11177446.9
(22) Anmeldetag: 13.11.2007
(51) Int. Cl.: A01N 59/16, A61L 27/54, A61L 29/16, A61L 31/16, B29C 45/00, C01G 39/00, C01G 41/00, C09C 1/00, C09D 5/14

(54) **Stoff mit antimikrobieller Wirkung**

(30) Priorität: 13.11.2006 AT 8052006 U
(62) Teilanmeldung aus: 07819790.2
(71) Anmelder: Guggenbichler, Joseph Peter, 6345 Kössen (AT)
(72) Erfinder: Eberhardt, Nico, 6600 Reutte (AT); Martinz, Hans-Peter, verstorben (AT); Wildner, Heiko, 87629 Füssen (DE); Guggenbichler, Joseph, Peter, Prof. Dr., 6345 Kössen (AT)
(74) Vertreter: Hofstetter, Alfons J.

(57) **Zusammenfassung**

Die Erfindung beschreibt die Verwendung eines anorganischen Stoffes, der in Kontakt mit einem wässrigen Medium Wasserstoff-Kationen bildet, die eine antimikrobielle Wirkung auslösen, wobei der Stoff Molybdän und/oder Wolfram enthält.

## Beschreibung

Die Erfindung betrifft die Verwendung eines Stoffes zur Erzielung einer antimikrobiellen Wirkung.

Mikroorganismen wie Bakterien und Pilze sind in unserem Lebensraum allgegenwärtig und besiedeln Oberflächen unterschiedlichster Art. Viele Mikroorganismen sind Krankheitserreger und ihre Verbreitung bzw. Bekämpfung spielt daher im Gesundheitswesen und in der Hygiene eine besondere Rolle. Gelangen solche Mikroorganismen in unseren Körper, können Sie die Ursache für lebensbedrohliche Infektionen sein. Zieht man sich eine solche Infektion in einem Krankenhaus zu, spricht man von einer nosokomialen Infektion.

Es wird davon ausgegangen, dass zur Beseitigung von durch nosokomiale Infektionen hervorgerufenen Schäden weltweit jährlich zweistellige Euro-Milliardenbeträge erforderlich sind. Die Bekämpfung krankheitserregender Mikroorganismen spielt daher im Gesundheitswesen und in der Hygiene eine besondere Rolle.

Neben der Abwehr bzw. der Abtötung unerwünschter Mikroorganismen, beispielsweise durch Antibiotika, erhalten präventive Maßnahmen, d.h. die Schaffung von für Mikroorganismen lebensfeindlichen Räumen, zunehmende Bedeutung. Unter diesen Präventivmaßnahmen hat die Verwendung von Silber als Zusatz in organischen und anorganischen Werkstoffen in den vergangenen Jahren rasch an Bedeutung gewonnen. Silberionen stören dabei wichtige Funktionen der Mikroorganismen. Es wird heute davon ausgegangen, dass Silberionen Enzyme blockieren und deren lebensnotwendige Transportfunktionen in der Zelle unterbinden. Weitere Effekte sind die Beeinträchtigung der Zellstrukturfestigkeit bzw. auch eine Schädigung der Membranstruktur. Diese Effekte können zur Zellschädigung bzw. zum Zelltod führen. Silber weist dabei ein sehr breites Wirkspektrum auch gegen multiresistente Keime auf. Für die Erzielung einer Langzeitwirkung reichen geringe Dosen aus. Man spricht dabei vom oligodynamischen Effekt. In machen Fällen werden noch organische Verbindungen zugesetzt, um die Wirksamkeit des Silbers zu erhöhen. Wichtig dabei ist immer, dass ausreichend Silberionen vorhanden sind. Es wird daher nanoskaliges Silberpulver, so genanntes Nanosilber, eingesetzt, um eine große Partikeloberfläche zu erzielen.

Silber besitzt in einem weiten Dosierspektrum keine toxischen Nebenwirkungen. Erst bei stark erhöhten Silberakkumulationen im Körper kann es zu Argyrie, einer irreversiblen schiefergrauen Verfärbung von Haut und Schleimhäuten kommen. Zudem können erhöhte Silberkonzentrationen Geschmacksstörungen, Störung der Geruchsempfindlichkeit sowie zerebrale Krampfanfälle verursachen.

Zudem ist zu erwähnen, dass generell die Wechselwirkung zwischen nanoskaligen Partikeln und dem menschlichen Organismus noch nicht hinreichend erforscht ist. Breite Untersuchungsprogramme wurden erst in jüngster Vergangenheit gestartet. Für viele Anwendungen ist die antimikrobielle Wirksamkeit von Silber nicht ausreichend. Die Wirksamkeit ist nur bis 0,25 molarer Kochsalzlösung gegeben. Darüber hinaus kommt es zur Bildung von Silberchlorid. Der wesentliche Nachteil bei der Verwendung von Nanosilber liegt in einer nicht befriedigenden Kostensituation. Dies liegt zum einen am hohen Silberpreis, zum anderen ist die Verarbeitung von Silber zu Nanopartikeln aufwendig. Ein weiteres Problem entsteht bei der Verarbeitung von Nanosilber, verursacht durch Agglomerat-, Aggregat- und Clusterbildung. Dadurch wird die aktive Oberfläche reduziert und in weiterer Folge auch die antimikrobielle Wirkung. Um dies zu verhindern, wird Nanosilber auf Partikeloberflächen eines Trägers, beispielsweise TiO₂, abgeschieden, was wiederum die Herstellkosten erhöht.

Es hat daher nicht an Versuchen gefehlt, bei anderen Metallen eine antimikrobielle, olygodynamische und desinfizierende Wirkung nachzuweisen. So zeigt auch Kupfer eine starke antimikrobielle Wirkung, jedoch bei zu hoher Zytotoxizität. Eine Recherche in der Internetenzyklopädie Wikipedia unter dem Suchbegriff Olygodynamie weist als Ergebnis aus, dass bisher dieser Effekt bei folgenden Metallen gefunden wurde, absteigend gereiht nach der Wirksamkeit: Quecksilber, Silber, Kupfer, Zinn, Eisen, Blei und Wismut. Auch Gold und Osmium, zwei edle und teure Metalle, zeigen diesen Effekt.

Für viele Anwendungen ist es jedoch erforderlich, dass neben einer ausreichenden antimikrobiellen Wirksamkeit der Wirkstoff keine Zytotoxizität und Thrombogenität aufweist, sowie allgemein biokompatibel ist. Wirkstoffe wie Quecksilber, Wismut oder Kupfer weisen diese Eigenschaften aufgrund ihrer hohen Zytotoxizität und nicht gegebenen Biokompatibilität nicht auf.

Eine Vielzahl von Patent- und Nicht-Patentliteratur beschäftigt sich mit der Herstellung und der Verwendung von Nanosilber. Nur vereinzelt werden weitere Metalle und anorganische Verbindungen beschrieben. So ist in der US 5 520 664 ein Katheter aus Kunststoff offenbart. Zur Erzielung einer antimikrobiellen Wirkung werden Atome durch Ionenimplantation eingebracht. Als antimikrobiell wirkende Metalle werden hier Silber, Chrom, Aluminium, Nickel, Wolfram, Molybdän, Platin, Iridium, Gold, Silber, Quecksilber, Kupfer, Zink und Cadmium erwähnt. In den Beispielen und besonderen Ausführungsformen sind jedoch nur Silber und Kupfer erwähnt.

Die JP 2001-54320 beschreibt einen Kunststoff, der 0,005 bis 1 Gew.% einer Mischung aus Molybdäntrioxid und Silberoxid enthält. Die Erfindung bezieht sich dabei auf eine Folie, welche aus einer antibakteriellen Harzkomponente sowie aus Komponenten besteht, die für das Trennwandmaterial von Reinräumen für die oberste Schicht von Bodenbelägen, Verkleidungen, Aktenmappen, Schreibtischunterlagen, Tischtüchern, Verpackungstüten, Textilwaren und dergleichen verwendet werden können. Die Problemstellung dabei ist, dass bei Einarbeitung eines anorganischen, antibakteriellen Wirkstoffs die Transparenz des Kunststoffes, beispielsweise von Vinylchloridharz, verloren geht. Der Verlust der Transparenz wird durch Zumischen von Oxid des sechswertigen Molybdäns vermieden. Es ist dieser Anmeldung weiters zu entnehmen, dass bei Überschreitung des Gewichtsverhältnisses Molybdäntrioxid zu Silberoxid von 95 : 5 keine antibakterielle Wirkung mehr erzielt werden kann. Es wird daher dem Molybdänoxid an sich keine antibakterielle Wirkung zugeschrieben. Ist das Verhältnis Molybdänoxid zu Silberoxid kleiner als 30 : 70, d.h. bei geringen Molybdänoxidanteilen, kommt es zu einer Verfärbung des Vinylchloridharzes.

In der JP 2001-040222 ist ein antimikrobieller Kunststoff beschrieben, der sowohl antimikrobiell wirkende organische, als auch metallische Komponenten enthält. Als antimikrobiell wirkende metallische Komponenten werden Silber, Platin, Kupfer, Zink, Nickel, Kobalt, Molybdän und Chrom erwähnt. In den Beispielen und bevorzugten Ausführungsformen werden wiederum nur Silber und Kupfer als wirksam beschrieben.

In der JP 2000-143369 ist eine Glasur für keramische Komponenten offenbart, die Silbermolybdat enthält. Es werden dabei 0,01 bis 1 % Silbermolybdat zugesetzt und in metallisches Silber umgewandelt. Durch den Zusatz von 10 bis 50 % Titanoxid wird die Wirkung verstärkt.

Auch durch photooxidativ wirkende Komponenten kann eine antimikrobielle Wirkung erzielt werden. Dadurch entstehen reaktive freie Radikale, die Mikroorganismen schädigen. So beschreibt die JP 11012479 einen antimikrobiellen Kunststoff, der eine organische und eine anorganische Komponente enthält. Beispielhaft für anorganische Komponenten werden metallische Partikel, wie Silber Zink und Kupfer, und weiters Verbindungen, wie Kalzium-Zink-Phosphat, Keramik, Glaspulver, Aluminium-Silikat, Titan-Zeolit, Apatit und Kalziumcarbonat erwähnt. Metalloxide wie Zinkoxid, Titanoxid oder Molybdänoxid wirken dabei als Katalysator für den photooxidativen Effekt. Aus der JP 11012479 ist daher zu entnehmen, dass die antimikrobielle Wirksamkeit nur dann erreicht wird, wenn der photooxidative Mechanismus auftritt; d.h. die Voraussetzung für die Wirksamkeit ist das Einwirken elektromagnetischer Strahlung.

Die Verfügbarkeit von kostengünstigen Werkstoffen mit antimikrobieller Wirksamkeit erlangt immer höhere Bedeutung. Diese Eigenschaften sind insbesondere dann wichtig, wenn sich viele Menschen auf engem Raum aufhalten oder wo hohe Ansprüche an die Hygiene gestellt werden, wie dies beispielsweise in Krankenhäusern, Arztpraxen, Pflegeheimen und öffentlichen Einrichtungen der Fall ist. Die Reduktion der nosokomialen Infektion ist dabei von besonderer Bedeutung. So wird geschätzt, dass es bei 0,5 % aller Hüftgelenks- und bei 2 bis 4 % aller Kniegelenksimplantate zu Infektionen kommt. Ein hohes Infektionsrisiko besteht insbesondere auch bei Kathetern. Daneben besteht auch ein Bedarf in vielen weiteren Anwendungsgebieten, die Ansiedlung und Vermehrung von Mikroorganismen zu steuern bzw. zu verhindern.

Außerdem ist neben der Verfügbarkeit eines Stoffes mit antimikrobieller Wirkung von weiterem Interesse, dass der antimikrobiell wirksame Stoff, wenn er in Verbundwerkstoffe, aus denen dann die entsprechende Artikel wie z.B. Katheter, Implantate, Filter, Rohre, Container, Kabel etc. hergestellt werden, eingearbeitet wird, nicht in seiner Wirksamkeit abgeschwächt wird.

Kunststoffe sind in der Regel preisgünstig in ihre Herstellung und einfach in der Handhabung. Sie sind somit bei vielen Anwendungen besonders bevorzugt. Dabei stellt sich jedoch die Problematik, dass für die verschiedenen Anwendungen unterschiedliche Kunststoffarten eingesetzt werden müssen, da die Eigenschaften wie z.B. Biegsamkeit bzw. Steifigkeit sowie Belastbarkeit von der Art des Kunststoffes abhängig ist. So eignet sich nicht jeder Kunststoff für jede Anwendung, z.B. Katheter oder Infusionsbeutel müssen nach wie vor eine gewisse Biegsamkeit aufweisen, im Gegensatz z.B. zu Implantaten oder Abfallbehältnissen. Folglich muss bei jeder verwendeten Kunststoffart getestet werden, ob die antimikrobiellen Stoffe in Verbindung mit dem entsprechenden Kunststoff ihre Wirksamkeit beibehalten bzw. wie diese entsprechend mit dem Kunststoffe eingesetzt werden müssen, um ihre Wirksamkeit zu erhalten. Dies führt jedoch zu aufwendigen und umfangreichen Testreihen und zwar für jede gewünschte Anwendung erneut, was wiederum zu höheren Herstellungskosten führt.

Es ist daher Aufgabe der vorliegenden Erfindung, einen Wirkstoff bereitzustellen, der eine hohe antimikrobielle Wirksamkeit, vergleichbar mit Nanosilber, aufweist. Für medizinische Anwendungen soll der Wirkstoff nur geringfügig zytotoxisch und thrombogen sein und zudem eine hohe allgemeine Biokompatibilität aufweisen. Zudem soll der Wirkstoff ein hohes Nutzen-Kosten-Verhältnis besitzen und günstige Verarbeitungseigenschaften aufweisen. Zudem ist vorteilhaft, wenn der Stoff nicht nur in Form nanoskaliger Partikel (Partikelgröße unter 100 nm), sondern auch in Form nicht lungengängiger Partikel (Partikelgröße größer 500 nm) bzw. auch in kompakter Form antimikrobiell wirkt. Außerdem ist es wünschenswert, dass man einen Verbundwerkstoff zur Verfügung stellt, der den antimikrobiell wirksamen Stoff enthält, der vielseitig einsetzbar ist, wobei der Stoff seine volle Wirkung im Verbundwerkstoff beibehält.

Diese vielschichtige Aufgabe ist dadurch gelöst, dass ein anorganischer Stoff verwendet wird, der in Kontakt mit einem wässrigen Medium die Bildung von Wasserstoff-Kationen bewirkt, zur Erzielung einer antimikrobiellen Wirkung, dadurch gekennzeichnet, dass der Stoff Molybdän und/oder Wolfram enthält.

Während bei den bis dato verfügbaren anorganischen Wirkstoffen der olygodynamische Effekt, d.h. die schädigende Wirkung von Metall-Kationen auf lebende Zellen genützt wird, wird bei der vorliegenden Erfindung die Bildung von Wasserstoff-Kationen, die eine Absenkung des pH-Werts in dem mit dem Stoff in Kontakt stehenden Medium bewirken, genutzt. Freie Protonen lagern sich dabei, auf Grund ihres sehr kleinen Radius, sofort an ein Wassermolekül unter Bildung von Oxoniumionen (H₃O⁺) an. Sofern die Konzentrationsverhältnisse es erlauben, kommt es zu Verknüpfungen der Oxoniumionen mit mehreren Wassermolekülen. Es werden daher als Wasserstoff-Kationen neben H⁺ auch die durch Reaktion des H⁺ mit Wasser gebildeten Kationen, sowie deren Hydrate bezeichnet. Neben dem Oxoniumion (H₃O⁺) sind dies das Zundelkation (H₅O₂⁺) und das Eigenkation (H₉O₄⁺).

So reagiert beispielsweise Molybdänoxid mit Wasser zu Molybdänsäure (H₂MoO₄), die sich wiederum mit H₂O zu H₃O⁺ und MoO₄⁻ oder MoO₄²⁻ umsetzt. Auch Wolframoxid bildet mit H₂O Wolframsäure (H₂WO₄), die sich mit H₂O zu H₃O⁺ und WO₄⁻ oder WO₄²⁻ umsetzt. Nach Arrhenius ist das Wasserstoffkation Träger der sauren Eigenschaften. Der pH-Wert ist der negativ dekadische Logarithmus des Zahlenwertes der Wasserstoffionen-Konzentration in Mol / Liter. Für eine reine neutrale Lösung von Wasser weisen die Wasserstoffionen und die OH⁻ (Hydroxid) Ionen den gleichen Wert auf (10⁻⁷ Mol/l) und der der pH-Wert beträgt 7. Bildet nun ein Stoff in Kontakt mit einem wässrigen Medium Wasserstoffkationen, so kommt es zu einer Erhöhung des Wasserstoffkationen-Wertes und damit wird das wässrige Medium sauer.

Es hat sich nun in überraschender Weise herausgestellt, dass in Kontakt mit einem wässrigen Medium Wasserstoff-Kationen bildende Stoffe eine ausgezeichnete antimikrobielle Wirksamkeit aufweisen. Ein wesentlicher Vorteil liegt auch darin, dass sich die Stoffe dabei praktisch nicht verbrauchen. Dies ist insbesondere dann der Fall, wenn der Stoff eine geringe Löslichkeit im wässrigen Medium aufweist. Bevorzugt liegt dabei die Löslichkeit bei kleiner 0,1 Mol / Liter. Die Löslichkeit von Molybdän- und Wolframoxid liegt bei kleiner 0,02 Mol / Liter. Der antimikrobielle Effekt ist daher zeitlich nahezu unbeschränkt vorhanden.

Das wässrige Medium kann nun beispielsweise Wasser, eine Lösung oder eine Suspension sein. Beispiele für eine Lösung sind die Körperflüssigkeit, für eine Suspension flüssiges Gewebe. Dabei reicht es aus, dass das wässrige Medium in Form eines dünnen Films auf der Stoffoberfläche vorliegt. Die erfinderische Wirkung wird bereits bei einer Dicke des Films im Nanometerbereich, wie es bei Adsorbat der Fall ist, erreicht. Der erfinderische Effekt tritt daher bereits auf, wenn sich der Stoff an Luft befindet. Durch die Bildung von Wasserstoffkationen kommt es dabei zu einer Absenkung des pH-Wertes auf typischer Weise < 6, bevorzugt < 5. Das dadurch erzeugte saure Milieu bewirkt eine Abtötung der Mikroorganismen.

Die Wirkung erfindungsgemäßer Stoffe wurde in breit angelegten Testreihen untersucht. Dabei wurden die antimikrobielle Wirkung, sowie zum Teil auch die Zytotoxizität und die Thrombogenität geprüft. Wie in den Beispielen dargelegt, sind Molybdän- und Wolfram-haltige Werkstoffe, die oberflächlich oxidiert sind oder in oxidischer Form vorliegen, besonders wirksam. Molybdän kann in unterschiedlichen Oxidationsstufen (VI, V, IV) auftreten, nimmt an Redoxprozessen teil und bildet dabei relativ schwache Komplexe mit physiologisch wichtigen Verbindungen. Obwohl Molybdän eine essentielle biochemische Rolle besitzt, verbindet es sich nicht ausreichend stark mit physiologisch wichtigen Verbindungen, um einen ernsthaften Blockade-Effekt auf metabolische Prozesse aufzuweisen. Es ist daher auch keine Giftigkeit für den menschlichen Organismus gegeben. Es ist davon auszugehen, dass Molybdän in Tieren und Pflanzen in der Form eines einfachen Molybdations [MoO₄]²⁻ aufgenommen und transportiert wird. Diese [MoO₄]²⁻ Anionen können die Zellmembrane durchdringen ohne dabei die Zelle zu schädigen. Es ist daher davon auszugehen, dass Molybdän nicht zytotoxisch ist. Zudem ist auch keine thrombogene Wirkung bekannt. Molybdän ist daher auch für medizinische Anwendungen geeignet. Auch Wolfram-haltige Werkstoffe zeigen eine hohe antimikrobielle Wirksamkeit. Über die Thrombogenität kann derzeit noch keine eindeutige Aussage getroffen werden, da erste Versuche auf eine gewisse thrombogene Wirkung schließen lassen. Ob dies eine intrinsische Eigenschaft des Wolframs ist oder vom Bearbeitungszustand abhängt, ist noch zu klären.

Außer bei Molybdän- und Wolfram-haltigen Werkstoffen wurde ein antimikrobieller Effekt, verbunden mit einer pH-Wert Absenkung, auch bei Nioboxid, Manganoxid und Siliziumkarbid festgestellt.

Bei der Charakterisierung der antimikrobiellen Wirksamkeit wurde auf eine Methode zurückgegriffen, die in folgenden Fachaufsätzen im Detail beschrieben ist:
- Fremdkörper-assoziierte Infektionen in der Intensivmedizin - Therapie und Prävention, J.P. Guggenbichler, Antibiotika Monitor 20 (3), 2004, S. 52 - 64
- Inzidenz und Prävention Fremdkörper-assiozierter Infektionen, J.P. Guggenbichler, Biomaterialien 5 (4) 2004, S 228 - 236.

Insbesondere die dort beschriebene Methode der Ausrollkultur hat sich zur Untersuchung der antimikrobiellen Wirkung gut bewährt. Dabei wird eine Wirkstoffprobe für eine bestimmte Zeit, beispielsweise 3 Stunden, in eine Keimsuspension gegeben. Es kommt zum oberflächlichen Anwachsen von Keimen. Nach dieser Zeit werden die Proben über eine so genannte Agarplatte gerollt und in eine sterile physiologische Kochsalzlösung gegeben. Dieser Vorgang wird mehrmals alle 3 Stunden wiederholt. Diese wiederholte Abrolltätigkeit in 3-stündigem Abstand gibt Aufschluss, ob und mit welchem Effizienzgrad eine keimreduzierende oder keimtötende Wirkung eintritt. Diese Methode lässt sich für die Untersuchung verschiedener Mikroben, Bakterien und Viren anwenden. Die Untersuchungen zum Wirkungsnachweis der erfindungsgemäßen Stoffe erfolgten getrennt für die Referenzstämme Pseudomonas aeroginosa, Escherichia coli und Staphylococcus aureus. Als Referenzwerkstoffe wurden Silber und Kupfer verwendet.

Die besten Ergebnisse konnten wie bereits erwähnt mit Stoffen erreicht werden, die Molybdän und Wolfram enthalten. Es ist dabei efindungswesentlich, dass im Grenzbereich zwischen dem Molybdän- oder Wolfram-basierenden Wirkstoff Molybdänoxid oder Wolframoxid entsteht. Tritt diese Oxidbildung in einem nicht hinreichenden Ausmaß oder entsprechenden Morphologie auf, ist keine antimikrobielle Wirkung vorhanden. Dies erklärt auch, warum Molybdän und Wolfram bis dato nicht als antimikrobiell wirksame Werkstoffe eingesetzt werden.

Durch thermische Voroxidation bei Temperaturen vorteilhafterweise von größer 300°C kann eine antimikrobielle Wirksamkeit eingestellt werden. Die Voroxidation kann auch chemisch oder elektrochemisch durchgeführt werden. Diese Voroxidation ist bei massiven Mo und W Proben erforderlich. Es hat sich dabei gezeigt, dass, vergleichsweise zu einem in situ gebildeten Oxidfilm, ein durch eine Glühbehandlung voroxidiertes Material antimikrobiell wirksamer ist. Eine Voroxidation ist insbesondere dann durchzuführen, wenn die Einsatzbedingungen keine hinreichende Oxidation auslösen. Entscheidend dabei ist weiters, dass der Oxidfilm eine große spezifische Oberfläche aufweist.

Neben Reinmolybdän und Reinwolfram sind auch die Verbindungen und Legierungen dieser Stoffe wirksam, die hinreichend stabil sind und oberflächlich einen Oxidfilm ausbilden. Zu den Molybdänverbindungen, die eine antimikrobielle Wirksamkeit aufweisen, zählen Molybdänkarbid, Molybdännitrid, Molybdänsilizid und Molybdänsulfid. Molybdän, Molybdänoxid und die zuvor genannten Stoffe sind kommerziell auch in sehr feiner Form mit Partikelgrößen nach Fisher von < 1 µm erhältlich. Bei den geeigneten Molybdänlegierungen sind Mo-0,1 bis 1Gew.%La₂O₃, Mo-0,5Gew.%Ti-0,08Gew.%Zr-0,01 bis 0,04Gew.%C, Mo-5 bis 50Gew.%Re und Mol,2Gew.%Hf-0,02 bis 0,15Gew.%C zu erwähnen. Diese Legierungen bilden an der Oberfläche einen antimikrobiell wirksamen Oxidfilm aus. Im Falle von Wolfram sind ebenfalls Wolframwerkstoffe wirksam, die in situ oder durch eine vorhergehende Glühung einen Oxidfilm ausbilden. Neben oxidiertem Reinwolfram sind die Oxide des Wolframs wirksam. Hier seien besonders Wolframblauoxid (WO_{2,84}) und WO₃ erwähnt. Auch die Wolframlegierungen W-0,1 bis 1Gew.%La₂O₃ und W-1 bis 26Gew.%Re weisen eine gute antimikrobielle Wirksamkeit auf. Von den möglichen Wolframverbindungen, die an der Oberfläche einen Oxidfilm ausbilden, eignen sich insbesondere Wolframkarbid, Wolframsilizid und Wolframsulfid. Während der antimikrobielle Effekt bei Silber nur in ausreichendem Maße auftritt, wenn Silber in sehr feinkörnigem Zustand vorliegt, weisen die erfindungsgemäßen Stoffe auch dann eine antimikrobielle Wirksamkeit auf, wenn sie in kompakter, dichter Form vorliegen. Die Versuche haben gezeigt, dass die Wirksamkeit noch verstärkt wird, wenn die Oberfläche vergrößert wird. Daher kann es für viele Anwendungen vorteilhaft sein, wenn der Stoff in poröser Form vorliegt.

Die Wirksamkeit ist auch gegeben, wenn der Stoff als Schicht oder Bestandteil einer Schicht vorliegt. Als besonders vorteilhaft haben sich Molybdänoxid- und Wolframoxidschichten, bzw. auch Molybdän- und Wolframschichten erwiesen, die in situ, oder wenn in situ keine ausreichende Oxidation eintritt, durch Voroxidation oxidiert sind. Die Schichten können auf Kunststoffen, Keramiken oder Metallen abgeschieden sein. Besonders geeignete Abscheideverfahren sind die thermische Verdampfung, das Sputtern, die chemische Gasphasenabscheidung, die Elektroabscheidung und die Lichtbogenverdampfung. Mittels chemischer Gasabscheidung können beispielsweise Molybdänoxidschichten durch die Zersetzung von Molybdänhexacarbonyl (Mo(CO)₆) bei Atmosphärendruck erzeugt werden. Auch metallorganische CVD (MOCVD) ist möglich. Als metallorganische Verbindung kann dabei beispielsweise Molybdänacetylacetonat (MoO₂(CH₃COCH₂COCH₂)₂) verwendet werden. Mit diesen metallorganischen Verbindungen lassen sich bei Temperaturen im Bereich von ca. 400 bis 500°C Molybdänoxidfilme herstellen, wobei neben MoO₃ auch Mo₉O₂₆ und Mo₄O₁₁ festzustellen sind. Die Korngröße von < 1 µm bei Schichtstärken im Bereich von einigen µm begünstigt die antimikrobielle Wirksamkeit. Auch durch Reaktivelektronenstrahlverdampfung lassen sich Molybdänoxid- und Wolframoxidfilme abscheiden. Auch diese Filme weisen eine sehr feinkörnige Struktur mit Poren in der Größe von 50 bis 100 nm auf.

Als besonders geeignete Abscheidemethoden sind weiteres noch Elektrophorese und Solgel-Prozesse zu erwähnen.

Werden die Schichten auf Metallen abgeschieden, so sind die gängigen Implantatwerkstoffe, wie Titan, Eisen und Kobalt und deren Legierungen, zu bevorzugen. Auch im Falle von keramischen Substratwerkstoffen ist bevorzugt von etablierten Werkstoffen wie ZrO₂ und Al₂O₃ auszugehen, deren Reinheit besser 99 Gew.% ist. Die Schichten können auch auf Glas oder Glaskeramik abgeschieden werden.

Wie bereits erwähnt steigt die Wirksamkeit, wenn der Stoff zum wässrigen Medium hin eine möglichst große Oberfläche aufweist. Besonders gute Resultate lassen sich erzielen, wenn die Schicht eine spongiöse porige Struktur mit einer Porengröße von 50 bis 900 µm aufweist. Solche porigen Strukturen lassen sich beispielsweise erzeugen, indem der antimikrobiell wirksame Stoff in Form eines Slurrys oder aus der Gasphase mit optionaler anschließender Glühung abgeschieden wird. Eine große Oberfläche kann auch erzielt werden, wenn die Schicht in Form inselartiger, im Wesentlichen nicht zusammenhängender Agglomerate vorliegt. Besonders vorteilhaft ist es, wenn diese inselartigen Agglomerate die Oberfläche des Substratwerkstoffs zu 40 bis 90 % bedecken. Die bevorzugte Größe der einzelnen Stoffagglomerate liegt unter 5 µm. Für viele Anwendungen kann es ausreichen, wenn der erfindungsgemäße Stoff in Pulverform verwendet wird. Entsprechend ist es vorteilhaft, wenn sehr feinkörniges Pulver verwendet wird, d.h. dass die Partikelgröße nach Fisher < 5 µm, bevorzugt kleiner 1 µm beträgt.

Die besten Resultate konnten mit metallischen Verbundwerkstoffen bzw. Verbundpulver erzielt werden. Der Verbundwerkstoff kann dabei auch in Form eines Verbundpulvers vorliegen. Diese metallischen Verbundwerkstoffe enthalten neben dem erfindungsgemäßen Stoff ein weiteres, chemisch edleres Metall. Die Wechselwirkung mit dem chemisch edleren Metall begünstigt dabei die Bildung von Wasserstoff-Kationen. Mischungen der erfindungsgemäßen Stoffe mit einem edleren Metall wirken auch dann antimikrobiell, wenn die Proben nicht voroxidiert sind.

Das chemisch edlere Metall ist dabei bevorzugt Silber, Kupfer, Zinn und deren Legierungen. Besonders vorteilhaft sind die metallischen Verbundwerkstoffe Mo-Ag, Mo-Cu, Mo-Sn, W-Ag, W-Cu und W-Sn. Der Mo bzw. W-Gehalt liegt dabei bevorzugt bei 10 bis 90 At.%, wobei die besten Resultate mit 30 bis 80 At.% erzielt werden konnten.

Die Verwendung von Silber ist dann vorteilhaft, wenn der Werkstoff nicht zytotoxisch und thrombogen sein darf. Bei vielen Anwendungen spielen diese Eigenschaften jedoch keine Rolle. Beispielhaft seien hier Einrichtungsgegenstände für Hygieneräume erwähnt. Hier kann anstelle des teuren Silbers auf Kupfer zurückgegriffen werden, das in seiner antimikrobiellen Wirksamkeit Silber übertrifft. Eine sehr hohe Wirksamkeit ist auch gegeben, wenn ein Mo-Cu oder W-Cu Pulver anderen Werkstoffen zugesetzt wird. Daneben kann der metallische Verbundwerkstoff auch in kompakter Form, als Schicht oder beispielsweise als poröser Formkörper vorliegen. Für kompakte Verbundwerkstoffe kann die Herstellung vorteilhafterweise durch Infiltrationstechniken erfolgen.

Des Weiteren kann der erfindungsgemäße Stoff für die Herstellung eines antimikrobiellen Kunststoffs eingesetzt werden.

Dabei ist besonders ein Verbundwerkstoff, der den erfindungsgemäßen Stoff enthält, von Interesse, wenn dieser Verbundwerkstoff ein oder mehrere Werkstoffe enthält, wobei mindestens ein Werkstoff davon eine Polymermatrix enthält, die aus eine vernetzbaren Polymermischung gebildet wird. Diese Polymermischung enthält bevorzugt ein ungesättigtes Polyolefin (A), das eine Gesamtmenge von Kohlenstoff-Kohlenstoff-Doppelbindung/1000 Kohlstoffatomen von mehr als 0.37 aufweist, bevorzugt.

Es hat sich herausgestellt, dass dieser Verbundwerkstoff vielseitig einsetzbar ist.

Die Verwendung des ungesättigten Polyolefins in der Polymermischung führt dazu, dass die Polyermischung vernetzbar wird. Dies geschieht bevorzugt durch die vorhandenen Doppelbindungen in der Polymermischung. Über diese Doppelbindungen kann man dann aufgrund der Anzahl an Kohlenstoff-Kohlenstoff-Doppelbindungen im Polyolefin, aber auch in der Polymermischung, den Grad der Vernetzung steuern. Der Grad der Vernetzung bestimmt aber die Flexibilität bzw. Steifigkeit des Polymers. So weisen Polymere mit einem hohen Vernetzungsgrad auch eine höhere Steifigkeit auf als Polymere mit einem niedrigeren Vernetzungsgrad. Daher kann der erfindungsgemäße Verbundwerkstoff in den unterschiedlichsten Verwendungen eingesetzt werden.

Bevorzugt ist außerdem, wenn die vernetzbare Polymermischung ein weitere Copolymer (B) enthält.

Die Bezeichung "Gesamtmenge von Kohlenstoff-Kohlenstoff-Doppelbindungen" in Verbindung mit dem Begriff "ungesättigten Polyolefin (A)" bezieht sich dabei auf Doppelbindung die von Vinyl-, Vinyliden- und/oder trans-Vinylen-Gruppen stammen. Die Menge jeder Art von Doppelbindungen wird dabei nach einem Verfahren bestimmt wie es im experimentellen Teil von EP 1 731 566 beschrieben ist.

Durch die Einführung der Doppelbindung können die Vernetzungseigenschaften der Polymermischung kontrolliert werden, so dass der gewünschte Vernetzungsgrad eingestellt werden kann.

So ist für die verschiedenen Anwendungen ein Gesamtgehalt von Kohlenstoff-Kohlenstoff-Doppelbindungen von wenigstens 0,40 pro 1000 Kohlenstoffatomen bevorzugt. Von besonderem Interesse ist ein Gehalt von 0,45 bis 0,80 pro 1000 Kohlenstoffatomen.

Des weiteren ist bevorzugt, dass der Gesamtgehalt von Vinyl-Gruppen im ungesättigten Polyolefin höher als 0,11 pro 1000 Kohlenstoffatomen ist. Der besonders bevorzugte Bereich liegt hier zwischen 0,15 bis 0,80 pro 1000 Kohlenstoffatomen, er kann aber auch höher liegen.

Es ist bekannt, dass zwei Arten von Vinyl-Gruppen in Polymeren vorkommen. Der eine Typ wird in einem Polymerisationsprozess mittels z.B. einer β-Sizissionsreaktion von sekundären Radikalen erzeugt oder ist das Resultat eines sogenannten Kettentransferagens. Der zweite Typ, und dieser wird in der vorliegende Erfindung bevorzugt, wird durch Polymerisation zwischen wenigstens einem Olefinmonomer und wenigstens einem polyungesättigten Monomer hergestellt.

Beide Arten von Vinyl-Gruppen können in der Polymermischung der vorliegenden Erfindung enthalten sein. Es ist jedoch bevorzugt, dass der Gehalt an Vinyl-Gruppen, die durch die Polymerisation zwischen wenigstens einem Olefinmonomer und wenigstens einem polyungesättigtem Monomer mindestens 0,03/1000 Kohlenstoffatomen beträgt. Bevorzugt ist ein Gehalt zwischen 0,06 bis 0,40/1000 Kohlenstoffatomen.

Das ungesättigte Polyolefin kann in der vorliegenden Erfindung sowohl unimodal als auch multimodal, z.B. bimodal sein und eine Dichte zwischen 0,860 und 0,960 g/cm³, bevorzugt zwischen 0,880 und 0,955 g/cm³, besonders bevorzugt zwischen 0,900 und 0,950 g/cm³ aufweisen.

Es ist außerdem bevorzugt, dass das ungesättigte Polyolefin aus einem Olefinmonomer, bevorzugt dabei ist Ethylen oder Propylen, und wenigstens einem polyungesättigtem Monomer durch Polymerisation hergestellt wird.

Die Polymerisation kann dabei nach jeder beliebigen bekannten Methode durchgeführt werden, bevorzugt ist jedoch eine radikalische Polymerisation bei hohem Druck, wie sie in WO93/0822 näher beschrieben wird, anzuwenden.

Es ist außerdem bevorzugt, dass das ungesättigte Polyolefin mindestens 60 Gew.% Ethylen-Monomer enthält. Stärker bevorzugt ist ein Gehalt von mindestens 70 Gew.%, besonders bevorzugt ein Gehalt von mindestens 80 Gew.% und ganz besonders bevorzugt von mindestens 90 Gew.%.

Bei den polyungesättigten Comonomeren handelt es sich bevorzugt um Diene. Besonderes bevorzugt sind Diene, die aus einer Gruppe ausgewählt worden sind bestehend aus:
- einem Monomer mit einer Kohlenstoffkette, die frei von Heteroatomen ist und mindestens acht Kohlenstoffatome enthält, wobei sich mindestens vier Kohlenstoffatome zwischen den nicht-konjugierten Doppelbindungen befinden und wenigstens eine dieser Doppelbindungen sich in terminaler Stellung befindet,
- aus einem Siloxan entsprechend der Formel I, 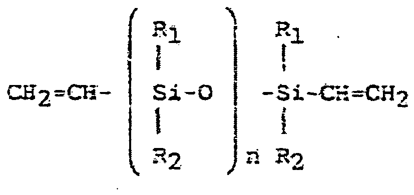 wobei R1 und R2 unterschiedliche oder gleiche Alkylgruppen sein können, bestehend aus 1-4 Kohlenstoffatomen und einer Alkoxy-Gruppe, die wiederum 1- 4 Kohlenstoffatome besitzt und n = 1 - 200 ist, und
- einem α,ω-Divinylether entsprechend der Formel II

   H₂C = CH-O-R-CH = CH₂

   wobei R ein -(CH₂)ₘ- O-, oder ein -(CH₂CH₂O)ₙ- oder -CH₂-C₆H₁₀-CH₂-O- ist und m = 2 bis 10 t und n = 1 bis 5 ist.

Die Diene können in allen denkbaren Kombinationen verwendet werden.

Die in der vorliegenden Erfindung verwendeten Diene und ihrer Herstellung sind in WO 93/08222, WO96/35732 und in WO 97/45465 näher beschrieben, worauf hier Bezug genommen werden soll.

Besonders bevorzugt ist es, dass die Diene aus einer Gruppe bestehend aus: 1,7 Octadien; 1,9-Decadien; 1,11-Dodecadien; 1,13-Tetradecadien; Tetramethyldivinyldisiloxan; Divinylpoly(diemethylsiloxan); und 1,4-Butadiendivinyl-ether oder einer Kombination daraus ausgewählt werden.

Neben den polyungesättigten Monomeren können weitere Comonomere in der Polymerisation verwendet werden, wie z.B. C₃ - C₂₀ Alpha-Olefine z.B. Propylen, 1-Buten, 1-Hexene und 1-Nonen, oder polare Comonomer wie z.B. Alkylacrylate, Alkylmethaacrylate und Vinylacetate.

Der Gehalt an polaren Monomeren im ungesättigten Polyolefin (A) beträgt jedoch weniger als 150 Mikromol, bevorzugt weniger als 125 Mikromol, ganz besonders bevorzugt weniger als 100 Mikromol.

Des weiteren ist bevorzugt, dass die Polymerischung ein weiteres Copolymer (B) enthält. Dieses Copolymer (B) ist vorzugsweise polar.

Daneben kann das polare Copolymer (B) auch wie das ungesättigte Polyolefin die oben beschrieben Verbindungen und damit die entsprechende Anzahl an Kohlenstoff-Kohlenstoff-Doppelbindungen enthalten. Die Vernetzbarkeit der Polymermischung wird dadurch noch einmal erhöht.

Dabei beträgt der Gehalt an Kohlenstoff-Kohlenstoff-Doppelbindungen im polaren Copolymer mindestens 0,15 pro 1000 Kohlenstoffatom. Bevorzugt ist ein Gehalt von 0,20 bis 0,35 pro 1000 Kohlenstoffatomen.

Insbesondere zeichnet sich aber das polare Copolymer dadurch aus, dass es polare Monomereinheiten enthält und zwar in einer Menge von mindestens 500 Mikromol pro Gramm polarem Copolymer, bevorzugt von 700 Mikromol, besonders bevorzugt von 900 Mikromol und ganz besonders bevorzugt von 1100 Mikromol pro Gramm polarem Copolymer.

Das polare Copolymer wird in einer Polymerisation aus einem Olefin, bevorzugt Ethylen, und einem polaren Comonomer hergestellt. Dabei kann auch wenigstens eines oder eine Mischung aus den oben beschriebenen polyungesättigten Monomeren zu gegen sein.

Bei dem polaren Comonomer handelt es sich bevorzugt um C₃ bis C₂₀ Monomere, die z.B. Hydroxyl-, Alkoxygruppen, Carbonyl-, Carboxy-, Estergruppen oder eine Mischung daraus enthalten.

Es ist weiterhin bevorzugt, dass die monomeren Einheiten aus eine Gruppe bestehend aus Alkylacrylaten, Alkylmethaacrylaten und Vinylacetaten ausgewählt werden.

Insbesondere bevorzugt ist es, dass es sich bei dem Comonomer um Comonomere ausgewählt aus der Gruppe bestehend aus C₁ bis C₆ Alkylakrylate, C₁ bis C₆ Alkylmethacrylate, oder Vinylacetate handelt. Als besonders bevorzugt gelten polare Monomere aus der Gruppe bestehend aus Alkylestern von Methacrylsäure wie z.B. Methyl, Ethyl oder Butylmethacrylat oder Vinylacetat. Der Acrylattyp ist dabei aufgrund seiner thermalen Beständigkeit bevorzugt.

Das polare Copolymer (B) sollte bevorzugt eine sogenannte "Melt Flow Rate" von MFR_{2.16/190 °C} 0,5 bis 70 g/10 min. stärker bevorzugt von 1 bis 55 g/10 min, ganz besonders bevorzugt von 1,5 bis 40 g/10 min haben.

Die vernetzbare Polymermischung wird vorzugsweise durch Mischen der beiden Komponenten - ungesättigtes Polyolefin (A) und polares Copolymer (B) hergestellt. Eine genaue Beschreibung des Herstellungsverfahrens der einzelnen Komponenten (A) und (B) ist in EP 1 731 566 zu finden.

Die vernetzbare Polymermischung enthält bevorzugt 5 bis 60 Gew.%, stärker bevorzugt 8 bis 50 Gew.%, besonders bevorzugt 10 bis 40 Gew.% und ganz besonders bevorzugt 15 bis 35 Gew.% des polaren Copolymers, basierend auf dem Gesamtgewicht an vernetzbarer Polymermischung.

Des weiteren ist bevorzugt, dass die vernetzbare Polymermischung einen Gesamtgehalt an Kohlenstoff-Kohlenstoff-Doppelbindungen pro 1000 Kohlenstoffatomen von mehr als 0,30 aufweist. Besonders bevorzugt ist ein Gesamtgehalt von mehr als 0,35; 0,40; 0,45, 0,50; 0,55 insbesondere von mehr als 0,60 an Kohlenstoff-Kohlenstoff-Doppelbindungen pro 1000 Kohlenstoff-atomen. Dabei basiert die Bestimmung auf den Gehalt an Vinyl-, Vinyliden und trans-Vinyliden Gruppen pro 1000 Kohlstoffatome sowohl des ungesättigten Polyolefins (A) als auch des polaren Copolymers (B).

Der Gehalt an Vinylgruppen liegt dabei bevorzugt zwischen 0,05 und 0,45 Vinylgruppen pro 1000 Kohlstoffatomen, stärker bevorzugt zwischen 0,10 und 0,40; besonders bevorzugt zwischen 0,15 und 0,35 pro 1000 Kohlstoffatomen.

Die Polymermatrix in der vorliegenden Erfindung entsteht durch die Vernetzung der beschriebenen vernetzbaren Polymermischung.

Dies geschieht vorzugsweise mit Hilfe eines Vernetzungsagens. Dieses Agens regeneriert Radikale und leitet somit die Vernetzungsreaktion ein. Als Agens bevorzugt sind Verbindungen die mindestens eine -O-O- oder eine -N=N- Bindung enthalten. Besonders bevorzugt ist die Verwendung von Peroxiden.

Als Peroxide eigenen sich z.B. Di-tert.-amylperoxid; 2,5-Di(tert-butylperoxy)-2,5-dimethyl-3-hexan; 2,5-Di(tert-butylperoxy)2,5-dimethylhexan, Tert.-butylcumyl-peroxid; Di(tert.-butyl)peroxid; Ducumylperoxid; Di(tert.-butylperoxid-isopropyl)benzol, Butyl-4,4-bis(tert.-butylperoxy)valerat; 1,1-Bis(tert.-butylperoxy)-3,3,5-trimethylcyclohexan; Tert.-Butylperoxybenzonat, Dienbenzoylperoxid.

Der antimikrobiell wirksame Stoff wird bevorzugt vor der Vernetzungsreaktion der Polymermischung hinzugemischt und bildet zusammen mit der Polymermatrix den Verbundwerkstoff.

Es ist dabei bevorzugt, dass der erfindungsgemäße Stoff im Kunststoff, insbesondere in der oben beschriebenen Polymermatrix, mit 0,1 bis 50 Vol.% eingearbeitet ist. In einer besonders vorteilhaften Ausführung sind dies 3 bis 15 Vol.%.

Die Vernetzungsreaktion findet unter den dafür üblichen Bedingungen statt, d.h. z.B. bei einer Temperatur von wenigstens 160 °C.

Es ist bevorzugt, dass die vernetze Polymermatrix eine Bruchdehnung eine sogenannte "hot set elongation" von weniger als 175%, stärker bevorzugt ist ein Wert von weniger als 100%, besonders bevorzugt von weniger als 90% aufweist, bestimmt nach der Methode IEC 60811-2-1. Der Wert der Bruchdehnung korreliert mit dem Grad der Vernetzung. Je geringer er ist, um so stärker ist die Polymermischung vernetzt.

Durch den Gehalt an Doppelbindungen und die Menge des Radikalinitiators kann wie bereits erwähnt der Vernetzungsgrad und damit die Steifigkeit der Polymermischung reguliert werden.

In vielen Anwendungen ist ein hochvernetztes Polyethylen bevorzugt.

Der erfindungsgemäße Verbundwerkstoff lässt sich gut durch Spritzgießen verarbeiten. Für die Herstellung des spritzgegossenen Verbundwerkstoffes wird bevorzugt ein in einem Extruder kompoundiertes Granulat verwendet, das gleichzeitig der Vernetzungsreaktion unterzogen wird. Im Gegensatz zur Herstellung von mit Silber-Nanopulver hergestellten Polymermatrixverbundwerkstoffen kann bei Verwendung der erfindungsgemäßen Stoffe auf einen Träger für den Wirkstoff, zur Vermeidung der Agglomerat- oder Clusterbildung verzichtet werden.

Die verwendete Polymermatrix im Verbundwerkstoff weist neben dem individuell einstellbaren Vernetzungsgrads und damit der Möglichkeit von verschieden Anwendungen, wie sie z.B. noch im Folgenden beschrieben werden, auch eine gute mechanische und thermische Stabilität auf.

Als Zusatzstoff zum Kunststoff bzw. der Polymermatrix verleihen Molybdänoxid, voroxidiertes Molybdän, Wolframoxid, voroxidiertes Wolfram, Mo-Cu, W-Cu, Mo-Ag und W-Ag dem Kunststoff bzw. dem Polymermatrixverbundwerkstoff eine ausgezeichnete antimikrobielle Wirksamkeit. Die besten Resultate konnten mit Mo-Cu, W-Cu, Mo-Ag und W-Ag erzielt werden. Auch hier ist wieder davon auszugehen, dass das chemisch edlere Metall die Oxidation des unedleren und damit in Folge die Erzeugung von Wasserstoffionen begünstigt. Wird als Zusatzstoff ein Mo-Cu, W-Cu, Mo-Ag oder W-Ag Verbundpulver verwendet, ist es wiederum wichtig, dass die Molybdän- bzw. Wolframphase und die Kupfer- bzw. Silberphase in sehr feiner Form vorliegen. Es kann beispielsweise ein durch einen Beschichtungsprozess hergestelltes Verbundpulver verwendet werden. Die Partikelgröße des Verbundpulvers liegt bevorzugt bei < 5 µm.

Der erfindungsgemäße Stoff kann auch im Verbund mit einem oder mehreren keramischen Werkstoffen vorliegen. Die Herstellung erfolgt beispielsweise durch Heißpressen. Als keramische Phase sind im besonderen Aluminiumoxid, Titanoxid, Siliziumoxid, Siliziumkarbid und Zirkonoxid geeignet. Um auf die üblichen Herstellmethoden und Bedingungen für Keramik zurückgreifen zu können, eignen sich erfindungsgemäße Zusatzstoffe, die in der höchsten Oxidationsstufe vorliegen, wie beispielsweise MoO₃ und WO₃. Daneben können noch metallisches Mo und W vorliegen.

Damit ergeben sich als geeignete Keramikverbundwerkstoffe folgende Materialkombinationen: Al₂O₃-MoO₃, Al₂O₃-WO₃, ZrO₂-MoO₃, ZrO₂-WO₃, Al₂O₃-Mo-MoO₃, Al₂O₃-W-WO₃, ZrO₂-MO-MoO₃, ZrO₂-W-WO₃, TiO₂-MoO₃, TiO₂-WO₃, TiO₂-Mo-MoO₃, TiO₂-W-WO₃, SiO₂-MoO₃, SiO₂-WO₃, SiO₂-Mo-MoO₃ und SiO₂-W-WO₃. Der vorteilhafte MoO₃ oder WO₃ Anteil beträgt dabei 0,001 bis 50 Mol.%. Das vorteilhafte Molverhältnis ZrO₂, Al₂O₃, TiO₂ oder SiO₂ zu MoO₃ oder WO₃ liegt bei 1 bis 100.

Aufgrund der hohen antimikrobiellen Wirksamkeit ergibt sich für die erfindungsgemäßen Stoffe eine Vielzahl von vorteilhaften Anwendungen. Dazu zählen Implantate und sonstige Geräte für die Medizintechnik. Bei den Implantaten wiederum können die erfindungsgemäßen Stoffe besonders vorteilhaft in Kathetern, Stents, Knochenimplantaten, Zahnimplantaten, Gefäßprothesen und Endoprothesen eingesetzt werden.

Zu den vorteilhaften Anwendungen im Bereich der Katheter zählen der Port- und der Blasenkatheter. Portkatheter umfassen dabei üblicherweise einen Topf mit einer Silikonmembran und einen angeschlossenen Schlauch. Der Topf besteht bis dato üblicherweise aus Kunststoff, kunststoffummanteltem Titan oder Keramik. Es kann nun der Katheter oder Teile des Katheters aus dem erfindungsgemäßen Stoff oder aus einem Werkstoff hergestellt werden, der den Stoff enthält. Es ist jedoch auch möglich, den Katheter oder Teile des Katheters mit einer erfindungsgemäßen Schicht zu versehen. Sehr gute Ergebnisse können erzielt werden, wenn der Topf aus einem Mo-Ag besteht, wobei der Ag-Gehalt bei 1 bis 40 Gew.% liegt. Dieser

Topf wird gemäß dem Stand der Technik wiederum mit Kunststoff ummantelt. Es ist weiterhin auch vorteilhaft, wenn der Kunststoff und/oder die Silikonmembran den Stoff enthalten.

Auch bei Luer-Lock-Verbindungen, Dreiweghähnen und Hahnenbänken können Probleme durch Bakterienkontamination auftreten und stellen damit bevorzugte Anwendungen für den erfindungsgemäßen Stoff dar.

Bei koronaren Stents ist es vorteilhaft, den erfindungsgemäßen Stoff durch ein Beschichtungsverfahren auf einen Stent aus einer Formgedächtnislegierung, beispielsweise Nitinol, aufzubringen. Auch bei Ureterstents kann der erfindungsgemäße Stoff vorteilhaft eingesetzt werden. Ureterstents werden üblicherweise aus Polyurethan oder Silikon gefertigt. Der erfindungsgemäße Stoff kann dabei dem Polymerwerkstoff zugesetzt werden oder wiederum oberflächlich als Schicht aufgebracht werden.

Knochenimplantate stehen mit dem Gewebswasser in Kontakt. Auch hier kann der erfindungsgemäße Stoff seine Wirkung entfalten. Vorteilhaft ist es dabei, den erfindungsgemäßen Stoff als Schicht aufzubringen. Ein Beispiel für ein Knochenimplantat ist das Hüftgelenk. Im Bereich des Gelenkkopfes ist es günstig, die Schicht glatt auszuführen, während der Implantatschaft mit einer spongiösen Beschichtung versehen werden kann. Da der erfindungsgemäße Stoff, wie bereits ausgeführt, in einfacher Form in einen Polymerwerkstoff eingebracht werden kann, eignet sich dieser auch zur Erzielung einer antimikrobiellen Wirkung bei Gefäßprothesen oder Herniennetzen. Zu den medizinisch technischen Verwendungen ist auch die Anwendung als Operationsbesteck zu zählen.

Außerdem kann der erfindungsgemäße Stoff bei jeglicher Art von Behältnissen, wie sie in der Medizin verwendet werden, eingesetzt werden. Insbesondere der Einsatz bei Nasensprayflaschen, wo eine hohe Gefahr der Kontamination mit Mikroorganismen besteht, ist die Verwendung des erfindungsgemäßen Stoffes von Vorteil.

Neben der rein medizinischen und veterinärmedizinischen Verwendbarkeit ist eine Vielzahl von Anwendungen im Hygienebereich möglich. So eignet sich der Stoff als Zusatzstoff für saugfähige Hygieneartikel oder Wundauflagen. Hygieneartikel und Wundauflagen enthalten Polymerfasern oder -gitter. In vorteilhafter Weise kann nun der erfindungsgemäße Stoff auf der Oberfläche der Fasern bzw. Gitter abgeschieden werden oder die Fasern bzw. Gitter können den Stoff enthalten.

Außerdem hat sich gezeigt, dass der erfindungsgemäß Stoff in Wundsprays - auch "Flüssig-Pflaster" genannt - wie sie heute handelsüblich erhältlich sind, als Zusatzstoff verwendet werden kann, um die antimikrobielle Wirkung dieser zu verstärken bzw. länger aufrecht zu erhalten, da diese häufig nur eine kurze antimikrobielle Wirkung zeigen. Bevorzugt hierbei ist die Verwendung des erfindungsgemäßen Stoffes, wenn dieser Molybdän enthält oder daraus besteht. Stärker bevorzugt ist wenn Molybdän bzw. dessen Verbindungen und Legierungen in einer Konzentration von 0,05 bis 1,0 Vol.%, besonders bevorzugt von 0,1 bis 0,5 Vol% in den Wundsprays verwendet wird.

Der erfindungsgemäße Stoff eignet sich auch als Zusatzstoff für Lacke, Beschichtungs- und Klebstoffe. Es hat sich dabei bewährt, wenn der Lack, Beschichtungs- oder Klebstoff 0,01 bis 70 Vol.% des Stoffes enthält. Der besonders bevorzugte Bereich liegt bei 0,1 bis 40 Vol.%. Für kostensensitive Produkte eignen sich als Zusatzstoffe insbesondere MoO₃ und/oder WO₃. Die bevorzugte Partikelgröße nach Fisher liegt dabei bei 0,5 bis 10 µm. Auf die Zugabe von Edelmetallen, zum Beispiel von Silber, kann dabei verzichtet werden. Wird jedoch eine besonders hohe Wirksamkeit gefordert, sind Zusatzstoffe auf der Basis W-Ag, W-Cu, Mo-Ag, Mo-Cu, Mo-Sn und W-Sn zu bevorzugen. Partikel, wiederum bevorzugt mit einer Größe nach Fisher von 0,5 bis 10 µm, können dabei über konventionelle Dispergiertechniken in zum Beispiel flüssige Lacksysteme, wie ZweiKomponenten-Polyurethanlacke, eingearbeitet werden.

Neben den Anwendungen im Medizin- und Hygienebereich kann der erfindungsgemäße Stoff auch als Zusatzstoff für ein Körperpflegemittel verwendet werden. Als vorteilhafte Produkte seien hier Salben, Seifen, Zahnpflegemittel, Zahncremen, Zahnprothese-Haftmittel, Zahnbürsten, Zwischenzahnreiniger und Zahnreinigungskaugummi erwähnt.

Des weiteren kann der erfindungsgemäße Stoff auch vorteilhaft als Zusatzstoff für einen Filter verwendet werden. Hier haben sich wiederum im besonderen Maße metallische Verbundwerkstoffe bewährt, die neben Wolfram oder Molybdän noch eine edlere Phase wie z.B. Silber, Kupfer oder Zinn enthalten. Der Filter kann dabei wiederum aus Polymerfäden bestehen, die den Stoff enthalten oder mit dem Stoff beschichtet sind.

Antibakteriell wirksame Stoffe werden derzeit bereits als Zusatzstoffe für Bekleidungsartikel und Schuheinlagen verwendet. Auch in diesem Anwendungsgebiet kann man die geringeren Kosten im Vergleich zu Nanosilber vorteilhaft nutzen. Die Polymerfaser kann dabei den Stoff enthalten oder der Stoff kann auf der Polymerfaser in abgeschiedener Form vorliegen.

Da der erfindungsgemäße Stoff in einfacher Form Lacken, Beschichtungsstoffen bzw. Kunststoffen beigemengt werden kann, eignen sich daraus hergestellte Produkte als Einrichtungsgegenstände, insbesondere für Hygieneräume.

Neben diesen Anwendungsgebieten eröffnen sich für den erfindungsgemäßen Stoff viele weitere Anwendungsgebiete, insbesondere für Produkte, die im häufigen Berührungskontakt mit Lebewesen stehen. Dazu zählen beispielsweise Schalter, Armaturen, Kreditkarten, Tastaturen, Handygehäuse, Münzen, Geldscheine, Türschnallen und Teile der Innenausstattung eines öffentlichen Verkehrsmittels. Eine weitere vorteilhafte Verwendung liegt in Bauteilen für Klimaanlagen. So eignet sich der erfindungsgemäße Stoff für Klimaanlagen in Verkehrsmittel, beispielsweise Automobilen. Die Kühlrippen, die üblicherweise aus einer Al-Legierung bestehen, können in vorteilhafter Weise mit dem erfindungsgemäßen Stoff beschichtet werden. Auch die Schächte von Klimaanlagen in Gebäuden können antimikrobiell ausgeführt werden, indem der Wirkstoff dem Schachtmaterial zugesetzt wird oder das Schachtmaterial mit diesem beschichtet wird. Auch Luftbefeuchter können mit entsprechenden antimikrobiellen Eigenschaften versehen werden.

Außerdem ist bevorzugt, den erfindungsgemäßen Stoff in Kabeln, insbesondere in Polyurethan enthaltenden Kabeln, zu verwenden.

Dies stellt nur eine beispielhafte Aufzählung möglicher vorteilhafter Anwendungen dar. Darüber hinaus kann der erfindungsgemäße Stoff überall dort eingesetzt werden, wo bereits Nanosilber Verwendung findet oder angedacht ist. Dabei ist zu berücksichtigen, dass je nach Anwendungsgebiet die Anforderungen bezüglich antimikrobieller Wirksamkeit, Thrombogenität und Zytotoxizität unterschiedlich sind.

Durch folgende Punkte wird die Erfindung weiter charakterisiert:
1. Verbundwerkstoff aus einem antimikrobiell wirksamen Stoff, der Molybdän und/oder Wolfram enthält und einem oder mehren Werkstoffen, dadurch gekennzeichnet, dass mindestens ein Werkstoff eine Polymermatrix enthält, die aus einer vernetzbaren Polymermischung gebildet wird, wobei die vernetzbare Polymermischung ein ungesättigtes Polyolefin (A), das eine Gesamtmenge von Kohlenstoff-Kohlenstoff-Doppelbindung/1000 Kohlstoffatomen von mehr als 0.37 aufweist, enthält.
2. Verbundwerkstoff nach Punkt 1, dadurch gekennzeichnet, dass die vernetzbare Polymermischung ein weiteres Copolymer (B) enthält.
3. Verbundwerkstoff nach Punkt 1 oder 2, dadurch gekennzeichnet, dass der Massengehalt des Stoffes 0,1 bis 50 Vol% im Verbundwerkstoff beträgt.
4. Verbundwerkstoff nach einem der Punkte 1 bis 3, dadurch gekennzeichnet, dass die Oberfläche des Stoffes zu mindestens partiell oxidiert ist.
5. Verbundwerkstoff nach einem der Punkte 1 bis 4, dadurch gekennzeichnet, dass der Stoff Molybdänoxid oder Wolframoxid ist.
6. Verbundwerkstoff nach einem der Punkte 1 bis 4, dadurch gekennzeichnet, dass der Stoff Molybdän, eine Molybdänlegierung und / oder eine Molybdänverbindung ist, wobei die Oberfläche eine Mo-Oxidschicht aufweist.
7. Verbundwerkstoff nach einem der Punkte 1 bis 4, dadurch gekennzeichnet, dass der Stoff Wolfram, eine Wolframlegierung und / oder eine Wolframverbindung ist, wobei die Oberfläche eine Wolfram-Oxidschicht aufweist.
8. Verbundwerkstoff nach Punkt 6, dadurch gekennzeichnet, dass die Molybdänlegierung Mo-0,1 bis 1 Gew.%La₂O₃, Mo-0,5Gew.%Ti-0,08Gew.%Zr-0,01 bis 0,04Gew.%C, Mo-5 bis 50Gew.%Re oder Mo1,2Gew.%Hf-0,02 bis 0,15Gew.%C ist.
9. Verbundwerkstoff nach Punkt 6, dadurch gekennzeichnet, dass die Molybdänverbindung Molybdänkarbid, Molybdännitrid, Molybdänsilizid und / oder Molybdänsulfid ist.
10. Verbundwerkstoff nach Punkt 7, dadurch gekennzeichnet, dass die Wolframlegierung W-0,1 bis 1 Gew.%La₂O₃ oder W- 1 bis 26 Gew.%Re ist.
11. Verbundwerkstoff nach Punkt 7, dadurch gekennzeichnet, dass die Wolframverbindung Wolframkarbid, Wolframnitrid, Wolframsilizid und / oder Wolframsulfid ist.
12. Verbundwerkstoff nach einem der Punkte 1 bis 11, dadurch gekennzeichnet, dass das ungesättigte Polyolefin (A) der vernetzbaren Polyolefinmischung aus einem Olefinmonomer und wenigstens einem polyungesättigtem Monomer durch Polymerisation hergestellt wird.
13. Verbundwerkstoff nach Punkt 12, dadurch gekennzeichnet, dass das Olefinmonomer Ethylen ist.
14. Verbundwerkstoff nach Punkt 12, dadurch gekennzeichnet, dass das polyungesättigte Monomer ein Dien ist.
15. Verbundwerkstoff nach Punkt 14, dadurch gekennzeichnet, dass die polyungesättigte Komponente entweder
   a) eine Kohlenstoffkette frei von Heteroatomen und mindestens aus 8 Kohlenstoffatomen und wenigstens 4 Kohlenstoffatomen zwischen den nicht konjugierten Doppelbindungen, wo von sich mindestens eine in terminaler Stellung befindet, besteht oder
   b) ein α,ω-Divinylsiloxan gemäß der Formel I 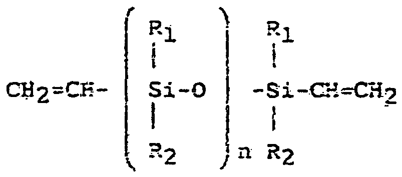 wobei R1 und R2 unterschiedliche oder gleiche Alkylgruppen sein können, bestehend aus 1-4 Kohlenstoffatomen und einer Alkoxy-Gruppe, die wiederum 1- 4 Kohlenstoffatome besitzt und n = 1 - 200 ist,
      oder
   c) ein α,ω-Divinylether gemäß der Formel II

      H₂C = CH-O-R-CH = CH₂

      wobei R ein -(CH₂)ₘ- O-, oder ein -(CH₂CH₂O)ₙ- oder -CH₂-C₆H₁₀-CH₂-O- ist und m = 2 bis 10 und n = 1 bis 5 ist,
   d) oder einer Mischung aus a), b), und/oder c) besteht.
16. Verbundwerkstoff nach den Punkten 1 bis 15, dadurch gekennzeichnet, dass das Copolymer (B) polar ist.
17. Verbundwerkstoff nach Punkt 16, dadurch gekennzeichnet, dass das polare Copolymer hergestellt wird durch Polymerisation aus einem Olefin und wenigstens einem polaren Copolymer.
18. Verwendung des Verbundwerkstoffes gemäß den Punkten 1 bis 17, in Artikeln bei denen mikrobielles Wachstum vermieden werden soll.
19. Verwendung gemäß Punkt 18, dadurch gekennzeichnet, dass es sich bei dem Artikel um ein medizinisches Produkt handelt.
20. Verwendung nach Punkt 19, dadurch gekennzeichnet, dass das Produkt ein Portkatheter ist, der einen Topf mit Silikonmembran und einen angeschlossenen Schlauch umfasst, wobei der Topf und/oder der Schlauch aus dem Verbundwerkstoff gemäß Punkte 1 bis 17 besteht.
21. Verwendung nach Punkt 20, dadurch gekennzeichnet, dass das Produkt eine Luer-Lock-Verbindung, ein Dreiweghahn und/oder eine Hahnbank ist.

Im Folgenden wird die Erfindung durch Beispiele näher erläutert.

Tabelle 1 enthält Angaben zur Probenfertigung.

Tabelle 2 gibt die Wirksamkeit gegen Staphylococcus aureus, Tabelle 3 gegen Escherichia coli und Tabelle 4 gegen Pseudomonas aeroginosa wieder.

### Beispiele

Die untersuchten Stoffe sind in Tabelle 1 wiedergegeben. Tabelle 1 enthält auch die Beschaffenheit der Ausgangsmaterialien und eine grobe Beschreibung der Probenherstellung. Bei den erfindungsgemäßen Proben W 02, W 03, W 04, W 05, Mo 02, Mo 03, Mo 04 und Mo 05 erfolgte der Pressvorgang in einer Matrizenpresse bei einem Pressdruck von ca. 250 MPa. Der Sintervorgang wurde bei diesen Proben in einem Wolframrohrofen bei einer Temperatur von 850°C / 60 Minuten unter reiner Wasserstoffatmosphäre durchgeführt. Unlegiertes Wolfram (Probe W 09) und unlegiertes Molybdän (Probe Mo 09) wurden bei 220 MPa isostatisch gepresst, bei einer Temperatur von 2250°C / 4 h bzw. 2100°C / 4h gesintert und anschließend einem Rundwalzvorgang unterzogen, wobei der Verformungsgrad ca. 70 % betrug.

Für die Herstellung der Polymermatrix-Verbundwerkstoffe wurde als Polymermatrix TransOptic, ein Acrylharz der Firma Bühler, verwendet, das üblicherweise für die Schliffherstellung Verwendung findet. Für das Abscheiden der Molybdänschichten (Proben SL 50, SL 51, SL 52) wurde das atmosphärische Plasmaspritzen angewandt. Die Schichtstärke betrug dabei ca. 100 µm und die Schichtdichte 85 % der theoretischen Dichte. Da der Beschichtungsvorgang an Luft durchgeführt wurde, betrug der Sauerstoffgehalt in der Schicht ca. 1,5 Gew.%. Der Sauerstoff liegt dabei hauptsächlich in Form von MoO₃ vor. Die Molybdänschichten wurden auf einer Titanlegierung (SL 50), Niob (SL 51) und einem intermetallischen Werkstoff ( SL 52) abgeschieden.

Als Vergleichsproben wurden unlegiertes Kupfer (Cu 01), unlegiertes Silber (SL 14), 20 Gew.% Kupferpulver eingebettet in eine Kunststoffmatrix (SL 20), 50 Gew.% Kupferpulver eingebettet in eine Kunststoffmatrix (SL 26), 20 Gew.% Silber eingebettet in eine Kunststoffmatrix (SL 21) und 50 Gew.% Silber eingebettet in eine Kunststoffmatrix (SL 27) verwendet. Weiters wurde zu Vergleichszwecken die antimikrobielle Wirksamkeit einer Reihe weiterer Werkstoffe, basierend auf Niob, Tantal und Titan, bestimmt.

Zur Untersuchung der antimikrobiellen Wirksamkeit wurde die bereits beschriebene Ausrollkultur verwendet. Die Untersuchungen erfolgten getrennt für Pseudomonas aeroginosa, Escherichia coli und Staphylococcus aureus. Die Wirkstoffprobe wurde dazu in eine Keimsuspension gegeben. Es kam zum oberflächlichen Anwachsen von Keimen. Nach 3, 6, 9 und 12 Stunden wurden die Proben über eine so genannte

Agarplatte gerollt und in eine sterile, physiologische Kochsalzlösung gegeben. Nach diesem Abrollvorgang wurde die Agarplatte fotografiert und bezüglich der keimreduzierenden bzw. keimtötenden Wirkung beurteilt. Die Fotos und die Bewertung der Wirksamkeit für Staphylococcus aureus sind in Tabelle 2, für Escherichia coli in Tabelle 3 und für Pseudomonas aeroginosa in Tabelle 4 wiedergegeben.

Es zeigt sich dabei, dass alle auf Wolfram oder Molybdän basierenden Stoffe in ihrer antimikrobiellen Wirksamkeit zumindest rein Silber in kompakter Form ebenbürtig sind oder zum Teil deutlich übertreffen. Als besonders wirksam haben sich dabei Proben, die neben Molybdän Silber oder Kupfer oder neben Wolfram Silber oder Kupfer enthalten, gezeigt.

Auch die Polymermatrixverbundwerkstoffe, die Molybdänoxid oder Wolframoxid enthalten, sind in ihrer antimikrobiellen Wirksamkeit als gut zu beurteilen. Die Verwendung von feinkörnigem Pulver, bevorzugt mit einer Partikelgröße nach Fisher von kleiner 5 µm ist dabei vorteilhaft.

Auf Tantal oder Niob basierende Proben zeigen mit Ausnahme der mit Cu versetzten Proben keine Wirksamkeit. Bei Ta-Cu und Nb-Cu kommt die hohe antimikrobielle Wirksamkeit des Kupfers, die jedoch mit Zytotoxizität einhergeht, zum Tragen.

Auch die Titan basierenden Vergleichsproben sind in ihrer Wirksamkeit als negativ zu beurteilen.

Die Versuche mit Polymermatrixwerkstoffen zeigten, dass man die Wirksamkeit über die Menge und die Partikelgröße des zugegebenen Molybdän- bzw. Wolframpulvers steuern kann. Je feiner das Molybdän- bzw. Wolframpulver ist, desto höher ist auch dessen Wirksamkeit (SL 33, SL 34). Dabei wirkt Molybdänoxidpulver stärker antimikrobiell als Molybdänmetallpulver (SL 22, SL 33).

Neben den hier aufgeführten Proben zeigten auch Nioboxid, Siliziumkarbid und Manganoxid eine antimikrobielle Wirkung, die auf eine Absenkung des pH-Wertes zurückzuführen ist.

Des Weiteren wurden auch erste Versuche zur Zytotoxizität durchgeführt. Es zeigte sich dabei, dass alle kupferhaltigen Werkstoff zytotoxisch sind. Auch erste Versuche zur Thrombogenität wurden durchgeführt. Silberhaltige Wolframlegierungen weisen eine im Vergleich zu silberhaltigen Molybdänlegierungen höhere Thrombogenität auf. Es ist hier jedoch einschränkend zu bemerken, dass auch die Oberflächengüte die Ergebnisse beeinflusst.

Zur Bestimmung des Wirkmechanismus wurden auch Versuche mit wasserlöslichen Molybdän- und Wolframsalzen durchgeführt. Dazu wurden Natriummolybdat (Na₂MoO₄) und Wolframmolybdat (Na₂WO₄) in eine Kunststoffmatrix eingebettet und dem oben beschriebenen Test zur Bestimmung der antimikrobiellen Wirksamkeit unterzogen. Dabei trat keine Absenkung des pH Wertes der physiologischen Kochsalzlösung ein. Die Proben waren antimikrobiell nicht wirksam. Es wurde in weiterer Folge der Gehalt an gelösten Elementen in der Kochsalzlösung nach einer Auslagerungszeit von 24 Stunden bestimmt. Wie zu erwarten liegt dieser Wert für die wasserlöslichen Verbindungen sehr hoch. So wurde beispielsweise für das Natriummolybdat ein Molybdängehalt in der Kochsalzlösung von 50 mg/l·cm² bestimmt. Vergleichsweise liegt dieser Wert für die antimikrobiell wirksamen Stoffe bei 0,1 (Probe SL 18), 0,4 (Probe SL 22) und 0,4 mg/l·cm² (Probe SL 24). Die antimikrobielle Wirksamkeit korreliert daher nicht mit dem Gehalt an Molybdän oder Wolfram in der physiologischen Kochsalzlösung.

Ähnliche Ergebnisse ergaben sich auch für Natriumwolframat. Hier betrug der Wolframgehalt in der Kochsalzlösung 324 mg/l·cm². Vergleichsweise wurden Werte von 0,1 für die Probe SL 17, 0,3 für die Probe SL 19 und 0,9 mg/l·cm² für die Probe SL 35 gemessen.

Bei silberhaltigen Werkstoffen, beispielsweise W 02 und W 03, wurde der Silbergehalt nach 24-stündigem Auslagern in physiologischer Kochsalzlösung bestimmt. Hier liegen die Werte für W 02 bei 28,6 und für W 03 bei 68,2 mg/l·cm².

Wie aus der Literatur bekannt wirkt Silber über die Bildung von Ag⁺ Ionen antimikrobiell. Die Wirksamkeit steigt mit zunehmender Ag⁺ Konzentration. Bei Molybdän und Wolfram konnte hingegen keine Abhängigkeit der antimikrobiellen Wirksamkeit vom Molybdän- bzw. Wolframgehalt in der physiologischen Kochsalzlösung ermittelt werden. Es ist daher davon auszugehen, dass Molybdän und Wolfram an sich nicht wirken. Es wurde daher in weiterer Folge auch der pH-Wert der physiologischen Kochsalzlösung nach Versuchende bestimmt. Für die antimikrobiell nicht wirksamen Werkstoffe wie Tantal, Tantal-5Ag, Tantal-20Ag, Niob, Niob-5Ag, Niob-20Ag, war der pH-Wert annähernd neutral. Auch Reinsilber bewirkt keine pH-Wert Absenkung.

Bei allen erfindungsgemäßen Proben konnte hingegen eine pH-Wert Absenkung ermittelt werden. So betrug der pH-Wert für W 09 4,8, für W 02 3,3, für W 03 3,1, für eine Probe Wolframkarbid mit 20 Gew.% Silber 5,3, für Mo 09 4,0, für Mo 02 3,9 und für Mo 03 3,8. Die Absinkung des pH-Wertes ist auf die Bildung von Oxoniumionen (H₃O⁺) zurückzuführen. Diese bilden sich aus der Umsetzung von H₂MoO₄ bzw. H₂WO₄ mit Wasser unter Abgabe von MoO₄⁻, MoO₄²⁻ bzw. WO₄⁻ oder WO₄²⁻.

H₂MoO₄ bzw. H₂WO₄ bilden sich wiederum aus der Umsetzung von MoO₃ bzw. WO₃ mit H₂O und / oder gelöstem Sauerstoff.

**Tabelle 1: Angaben zur Probenfertigung - Teil 1 von 3.**

| Material [Gew.-%] | Bemerkung | Ausgangsmaterialien | Probenherstellung |
|---|---|---|---|
| (Bezeichnung) | | | |
| Unlegiertes Wolfram oxidiert | Efindungsgemäß | ■ W- Pulver: Fisher-Korngröße 4,0µm | Pressen → Sintern → Umformung →Mechanische Bearbeitung → Oxidation (dichter Werkstoff) |
| (W_09) | | | |
| W-5Ag | Erfindungsgemäß | ■ W- Pulver: Fisher-Korngröße 4,0µm | Mischen → Pressen → Sintern → Mechanische Bearbeitung (poröser Werkstoff: 60 - 70% der theoretischen Dichte) |
| (W_02) | | ■ Ag- Pulver: Fisher-Korngröße 1,0µm | |
| W-20Ag | Erfindungsgemäß | ■ W- Pulver: Fisher-Korngröße 4,0µm | Mischen → Pressen → Sintern → Mechanische Bearbeitung (poröser Werkstoff: 60 - 70% der theoretischen Dichte) |
| (W_03) | | ■ Ag- Pulver: Fisher-Korngröße 1,0µm | |
| W-5Cu | Erfindungsgemäß | ■ W- Pulver: Fisher-Korngröße 4,0µm | Mischen → Pressen Sintern → Mechanische Bearbeitung (poröser Werkstoff: 60 - 70% der theoretischen Dichte) |
| (W_04) | | ■ Cu- Pulver: Fisher-Korngröße 6,9µm | |
| W-20Cu | Erfindungsgemäß | ■ W- Pulver: Fisher-Korngröße 4,0µm | Mischen → Pressen → Sintern → Mechanische Bearbeitung (poröser Werkstoff: 60 - 70% der theoretischen Dichte) |
| (W_05) | | ■ Cu- Pulver: Fisher-Korngröße 6,9µm | |
| Kunststoffmatrix + 20W- Pulver | Efindungsgemäß | ■ W- Pulver: Fisher-Korngröße 4,0µm | Mischen → Pressen → Mechanische Bearbeitung |
| (SL_17) | | ■ Kunststoffmatrix: TransOptic- Pulver von der Firma Buehler GmbH (Acrylharz) | |
| Kunststoffmatrix + 50W- Pulver | Erfindungsgemäß | ■ W- Pulver: Fisher-Korngröße 4,0µm | Mischen → Pressen → Mechanische Bearbeitung |
| (SL_23) | | ■ Kunststoffmatrix: TransOptic- Pulver von der Firma Buehler GmbH (Acrylharz) | |
| Kunststoffmatrix + 20(W2OAg)-Pulver | Erfindungsgemäß | ■ W- Pulver: Fisher-Korngröße 4,0µm | Mischen → Pressen → Mechanische Bearbeitung |
| | | ■ Ag- Pulver: Fisher-Korngröße 1,0µm | |
| (SL_19) | | ■ Kunststoffmatrix: TransOptic- Pulver von der Firma Buehler GmbH (Acrylharz) | |
| Kunststoffmatrix + 50(W20Ag)- | Erfindungsgemäß | ■ W- Pulver: Fisher-Korngröße 4,0µm | Mischen → Pressen → Mechanische Bearbeitung |
| | | ■ Ag- Pulver: Fisher-Korngröße 1,0µm | |
| Pulver (SL_25) | | ■ Kunststoffmatrix: TransOptic- Pulver von der Firma Buehler GmbH (Acrylharz) | |
| Kunststoffmatrix | Erfindungsgemäß | ■ W03- Pulver: Fisher-Korngröße 12µm | Mischen → Pressen → Mechanische Bearbeitung |
| + 50W03- Pulver (SL_35) | | ■ Kunststoffmatrix: TransOptic- Pulver von der Firma Buehler GmbH (Acrylharz) | |
| Unlegiertes Molybdänoxidiert | Erfindungsgemäß | ■ Mo- Pulver: Fisher-Korngröße 3,8µm | Pressen → Sintern → Umformung → Mechanische Bearbeitung → Oxidation (dichter Werkstoff) |
| (Mo_09) | | | |
| Mo-5Ag | Erfindungsgemäß | ■ Mo- Pulver: Fisher-Korngröße 3,8µm | Mischen → Pressen → Sintern → Mechanische Bearbeitung (poröser Werkstoff: 60 - 70% der theoretischen Dichte) |
| (Mo_02) | | ■ Ag- Pulver: Fisher-Korngröße 1,0µm | |
| Mo-20Ag | Erfindungsgemäß | ■ Mo- Pulver: Fisher-Korngröße 3,8µm | Mischen → Pressen → Sintern → Mechanische Bearbeitung (poröser Werkstoff: 60 - 70% der theoretischen Dichte) |
| (Mo_03) | | ■ Ag- Pulver: Fisher-Korngröße 1,0µm | |
| Mo-5Cu | Erfindungsgemäß | ■ Mo- Pulver: Fisher-Korngröße 3,8µm | Mischen → Pressen → Sintern → Mechanische Bearbeitung (poröser Werkstoff: 60 - 70% der theoretischen Dichte) |
| (Mo_04) | | ■ Cu- Pulver: Fisher-Korngröße 6,9µm | |

**Tabelle 1: Angaben zur Probenfertigung - Teil 2 von 3.**

| Material [Gew.-%] | Bemerkung | Ausgangsmaterialien | Probenherstellung |
|---|---|---|---|
| (Bezeichnung) | | | |
| Mo-20Cu | Erfindungsgemäß | ■ Mo- Pulver: Fisher-Korngröße 3,8µm | Mischen → Pressen → Sintern → Mechanische Bearbeitung (poröser Werkstoff: 60 - 70% der theoretischen Dichte) |
| (Mo_05) | | ■ Cu- Pulver: Fisher-Korngröße 6,9µm | |
| Kunststoffmatrix + 20Mo- Pulver | Erfindungsgemäß | ■ Mo- Pulver: Fisher-Korngröße 3,8µm | Mischen → Pressen → Mechanische Bearbeitung |
| (SL_16) | | ■ Kunststoffmatrix: TransOptic- Pulver von der Firma Buehler GmbH (Acrylharz) | |
| Kunststoffmatrix + 50Mo- Pulver | Erfindungsgemäß | ■ Mo- Pulver: Fisher-Korngröße 3,8µm | Mischen → Pressen → Mechanische Bearbeitung |
| (SL_22) | | ■ Kunststoffmatrix: TransOptic- Pulver von der Firma Buehler GmbH (Acrylharz) | |
| Kunststoffmatrix + 20(Mo20Ag)-Pulver | Erfindungsgemäß | ■ Mo- Pulver: Fisher-Korngröße 3,8µm | Mischen → Pressen → Mechanische Bearbeitung |
| | | ■ Ag- Pulver: Fisher-Korngröße: 1,0µm | |
| (SL_18) | | ■ Kunststoffmatrix: TransOptic- Pulver von der Firma Buehler GmbH (Acrylharz) | |
| Kunststoffmatrix + 50(Mo20Ag)-Pulver | Erfindungsgemäß | ■ Mo- Pulver: Fisher-Korngröße 3,8µm | Mischen → Pressen → Mechanische Bearbeitung |
| | | ■ Ag- Pulver: Fisher-Korngröße: 1,0µm | |
| (SL_24) | | ■ Kunststoffmatrix: TransOptic- Pulver von der Firma Buehler GmbH (Acrylharz) | |
| Kunststoffmatrix + 50MO₂- Pulver | Erfindungsgemäß | ■ MoO₂- Pulver: Fisher-Korngröße 3,6µm | Mischen → Pressen → Mechanische Bearbeitung |
| (SL_33) | | ■ Kunststoffmatrix: TransOptic- Pulver von der Firma Buehler GmbH (Acrylharz) | |
| Kunststoffmatrix+ 50MO₃- Pulver | Erfindungsgemäß | ■ MoO₃- Pulver: Fisher-Korngröße 15,9µm | Mischen → Pressen → Mechanische Bearbeitung |
| (SL_34) | | ■ Kunststoffmatrix: TransOptic- Pulver von der Firma Buehler GmbH (Acrylharz) | |
| Ti-46.5Al-4(Cr,Nb,Ta,B) beschichtet mit Molybdän | Erfindungsgemäß | ■ Schmelzingot | Strangpressen → Mechanische Bearbeitung → Mo-Beschichtung mittels Atmosphärischen Plasmaspritzen |
| (SL_50) | | | |
| Unlegiertes Niob beschichtet mitMolybdän | Erfindungsgemäß | ■ Nb- Pulver: Fisher-Korngröße 4,7µm | Pressen → Sintern → Umformung → Mechanische Bearbeitung → Mo-Beschichtung mittels Atmosphärischen Plasmaspritzen |
| (SL_51) | | | |
| Ti-6A1-4V-2Ag beschichtet mit Molybdän | Erfindungsgemäß | ■ Schmelzingot | Mechanische Bearbeitung → Mo-Beschichtung mittels Atmosphärischen Plasmaspritzen |
| (SL_52) | | | |
| Unlegiertes Tantal | Nicht erfindungsgemäß | ■ Ta- Pulver: Fisher-Korngröße 11,0µm | Pressen → Sintern → Umformung → Mechanische Bearbeitung (dichter Werkstoff) |
| (Ta_01) | | | |
| Ta-5Ag | Nicht erfindungsgemäß | ■ Ta- Pulver: Fisher-Korngröße 11,0µm | Mischern → Pressen → Sintern → Mechanische Bearbeitung (poröser Werkstoff: 60 - 70% der theoretischen Dichte) |
| (Ta_02) | | ■ Ag- Pulver: Fisher-Korngröße 1,0µm | |
| Ta-20Ag | Nicht erfindungsgemäß | ■ Ta- Pulver: Fisher-Korngröße 11,0µm | Mischen → Pressen → Sintern → Mechanische Bearbeitung (poröser Werkstoff: 60 - 70% der theoretischen Dichte) |
| (Ta_03) | | ■ Ag- Pulver: Fisher-Korngröße 1,0µm | |
| Ta-5Cu | Nicht erfindungsgemäß | ■ Ta- Pulver: Fisher-Korngröße 11,0µm | Mischen → Pressen → Sintern → Mechanische Bearbeitung (poröser Werkstoff: 60 - 70% der theoretischen Dichte) |
| (Ta_04) | | ■ Cu- Pulver: Fisher-Korngröße 6,9µm | |

**Tabelle 1: Angaben zur Probenfertigung - Teil 3 von 3.**

| Material [Gew.-%] | Bemerkung | Ausgangsmaterialien | Probenherstellung |
|---|---|---|---|
| (Bezeichnung) | | | |
| Ta-20Cu | Nicht erfindungsgemäß | ■ Ta- Pulver: Fisher-Korngröße 11,0µm | Mischen → Pressen Sintern → Mechanische Bearbeitung (poröser Werkstoff: 60 - 70% der theoretischen Dichte) |
| (Ta_05) | | ■ Cu- Pulver: Fisher-Korngröße 6,9µm | |
| Unlegiertes Niob | Nicht erfindungsgemäß | ■ Nb- Pulver: Fisher-Korngröße 4,7µm | Pressen → Sintern → Umformung → Mechanische Bearbeitung (dichter Werkstoff) |
| (Nb_01) | | | |
| Nb-5Ag | Nicht erfindungsgemäß | ■ Nb- Pulver: Fisher-Korngröße 4,7µm | Mischen → Pressen → Sintern → Mechanische Bearbeitung (poröser Werkstoff: 60 - 70% der theoretischen Dichte) |
| (Nb_02) | | ■ Ag- Pulver: Fisher-Korngröße 1,0µm | |
| Nb-20Ag | Nicht erfindungsgemäß | ■ Nb- Pulver: Fisher-Korngröße 4,7µm | Mischen → Pressen → Sintern → Mechanische Bearbeitung (poröser Werkstoff: 60 - 70% der theoretischen Dichte) |
| (Nb_03) | | ■ Ag- Pulver: Fisher-Korngröße 1,0µm | |
| Nb-5Cu | Nicht erfindungsgemäß | ■ Nb- Pulver: Fisher-Korngröße 4,7µm | Mischen → Pressen → Sintern → Mechanische Bearbeitung (poröser Werkstoff: 60 - 70% der theoretischen Dichte) |
| (Nb_04) | | ■ Cu- Pulver: Fisher-Korngröße 6,9µm | |
| Nb-20Cu | Nicht erfindungsgemäß | ■ Nb- Pulver: Fisher-Korngröße 4,7µm | Mischen → Pressen → Sintern → Mechanische Bearbeitung (poröser Werkstoff: 60 - 70% der theoretischen Dichte) |
| (Nb_05) | | ■ Cu- Pulver: Fisher-Korngröße 6,9µm | |
| Ti-6Al-4V-2Ag | Nicht erfindungsgemäß | ■ Schmelzingot | Mechanische Bearbeitung |
| (IM-Ti_01) | | | |
| Ti-46.5Al-4(Cr,Nb,Ta,B) | Nicht erfindungsgemäß | ■ Schmelzingot | Strangpressen → Mechanische Bearbeitung |
| (IM-TiAl_01) | | | |
| Unlegiertes Kupfer | Stand der Technik | ■ Umgeformter Cu- Stab | Mechanische Bearbeitung |
| (Cu_01) | | | |
| Unlegiertes Silber | Stand der Technik | ■ Umgeformtes Ag- Rohr | Mechanische Bearbeitung |
| (SL_14) | | | |
| Kunststoffmatrix + 20Cu- Pulver | Stand der Technik | ■ Cu- Pulver: Fisher-Korngröße 6,9µm | Mischen → Pressen → Mechanische Bearbeitung |
| (SL_20) | | ■ Kunststoffmatrix: TransOptic- Pulver von der Firma Buehler GmbH (Acrylharz) | |
| Kunststoffmatrix + 50Cu- Pulver | Stand der Technik | ■ Cu- Pulver: Fisher-Korngröße 6,9µm | Mischen → Pressen → Mechanische Bearbeitung |
| (SL_26) | | ■ Kunststoffmatrix: TransOptic- Pulver von der Firma Buehler GmbH (Acrylharz) | |
| Kunststoffmatrix + 20Ag- Pulver | Stand der Technik | ■ Ag- Pulver: Fisher-Korngröße 1,0µm | Mischen → Pressen → Mechanische Bearbeitung |
| (SL_21) | | ■ Kunststoffmatrix: TransOptic- Pulver von der Firma Buehler GmbH (Acrylharz) | |
| Kunststoffmatrix + 50Ag- Pulver | Stand der Technik | ■ Ag- Pulver: Fisher-Korngröße 1,0µm | Mischen → Pressen → Mechanische Bearbeitung |
| (SL_27) | | ■ Kunststoffmatrix: TransOptic- Pulver von der Firma Buehler GmbH (Acrylharz) | |

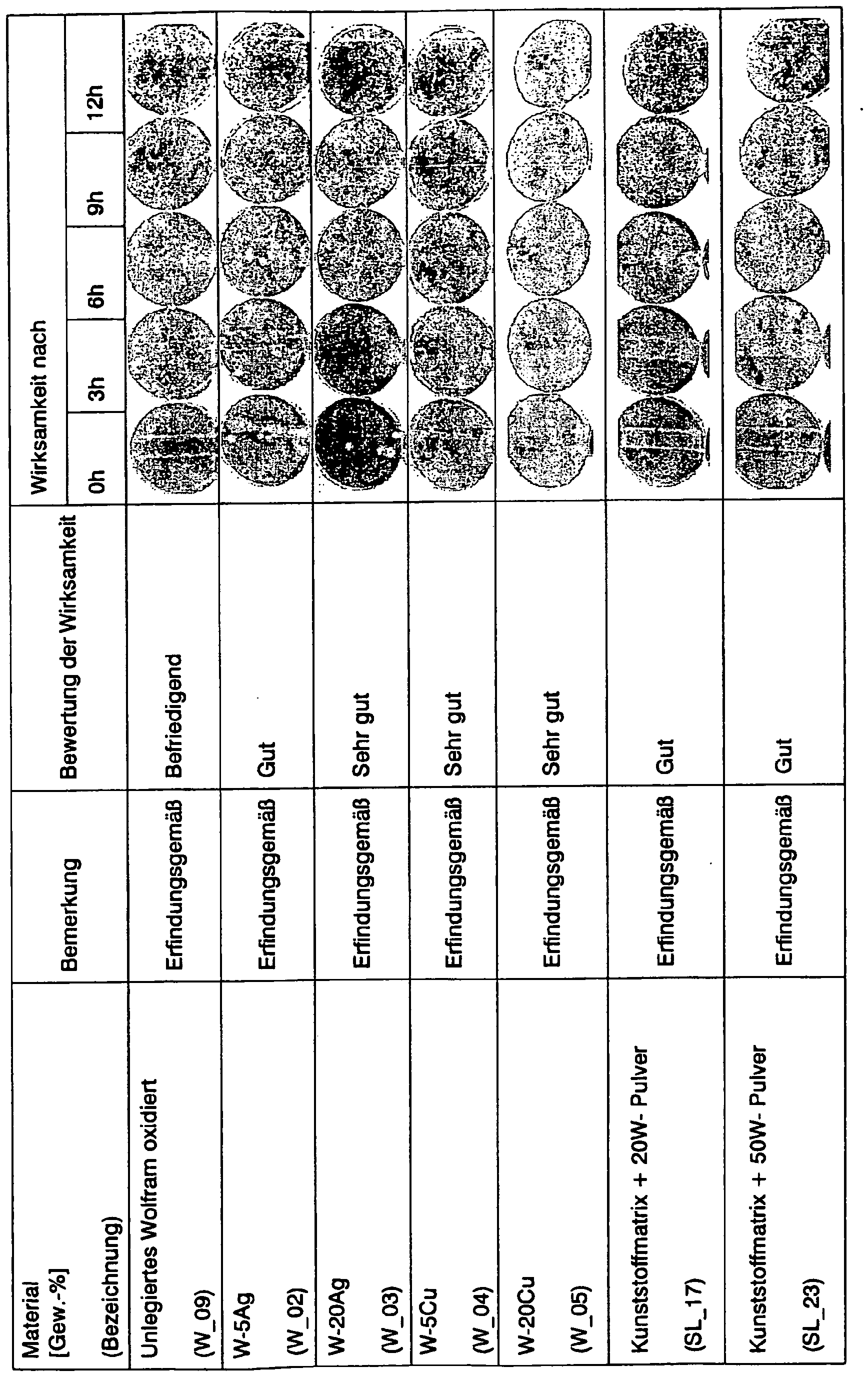

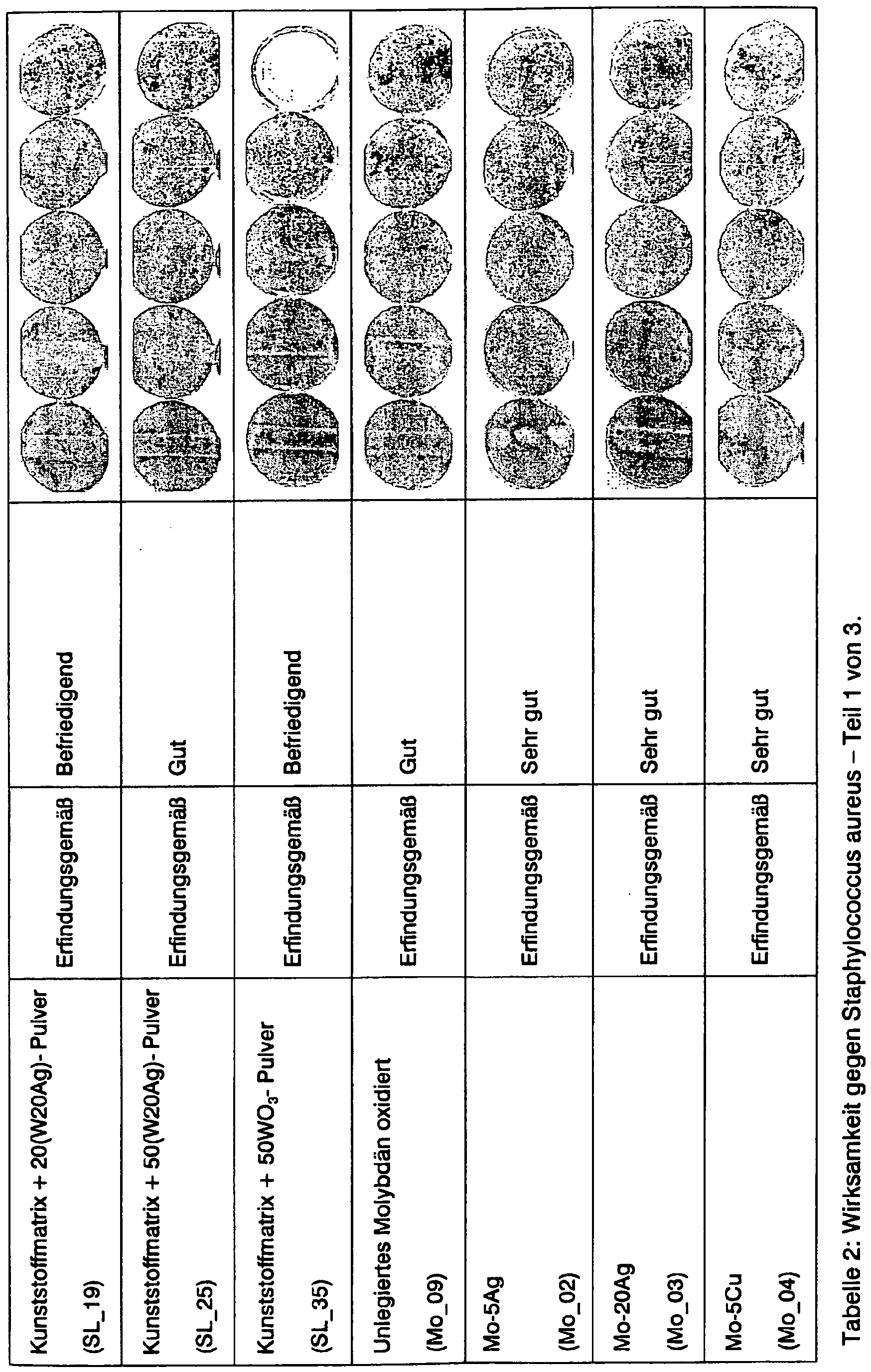

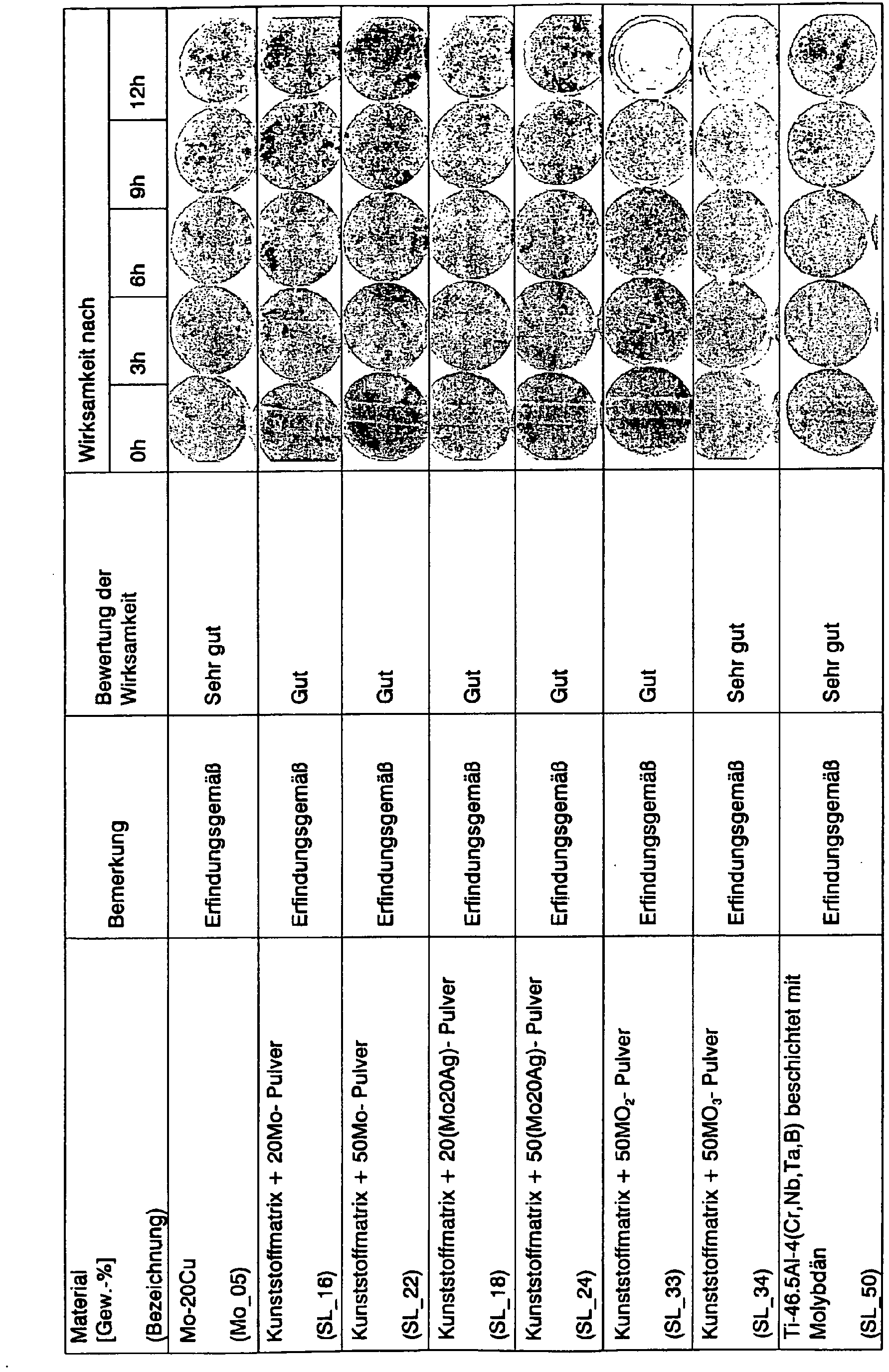

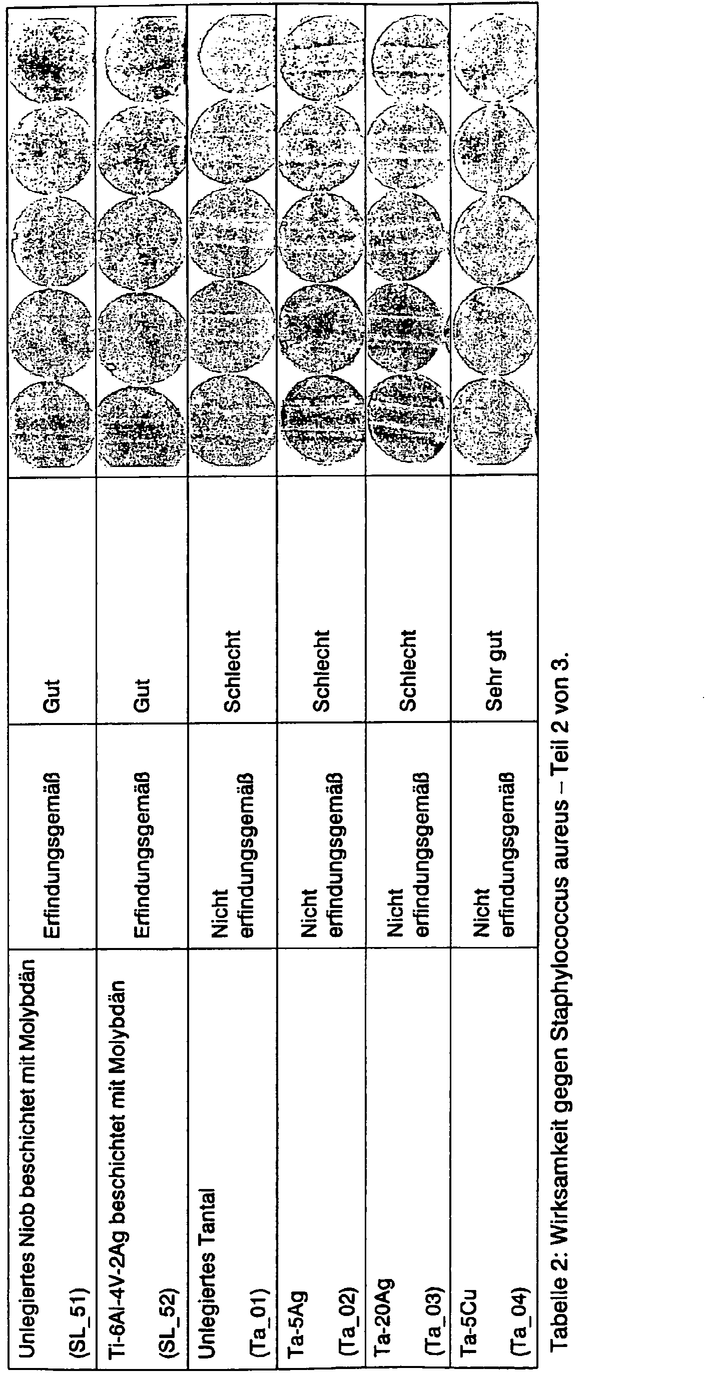

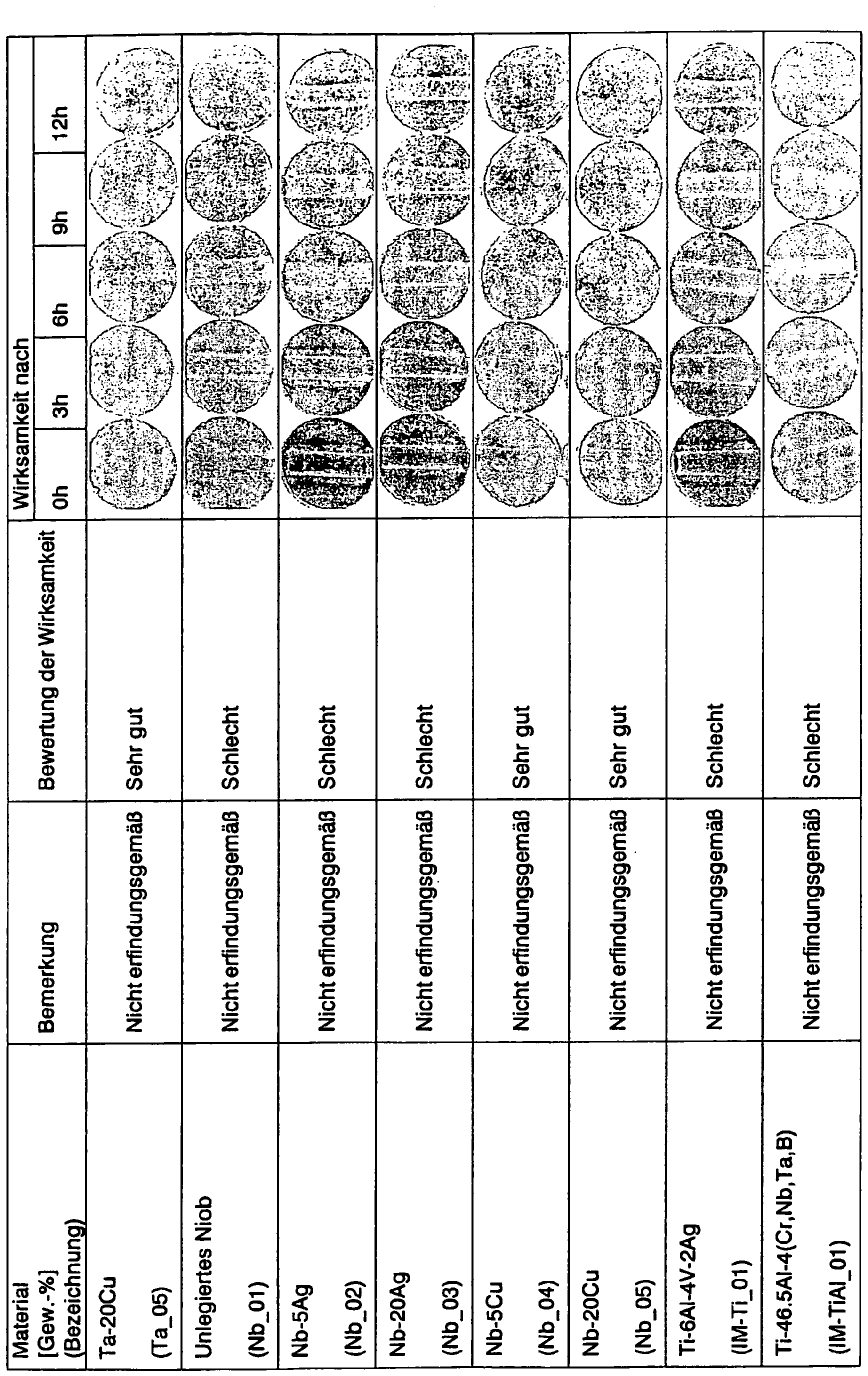

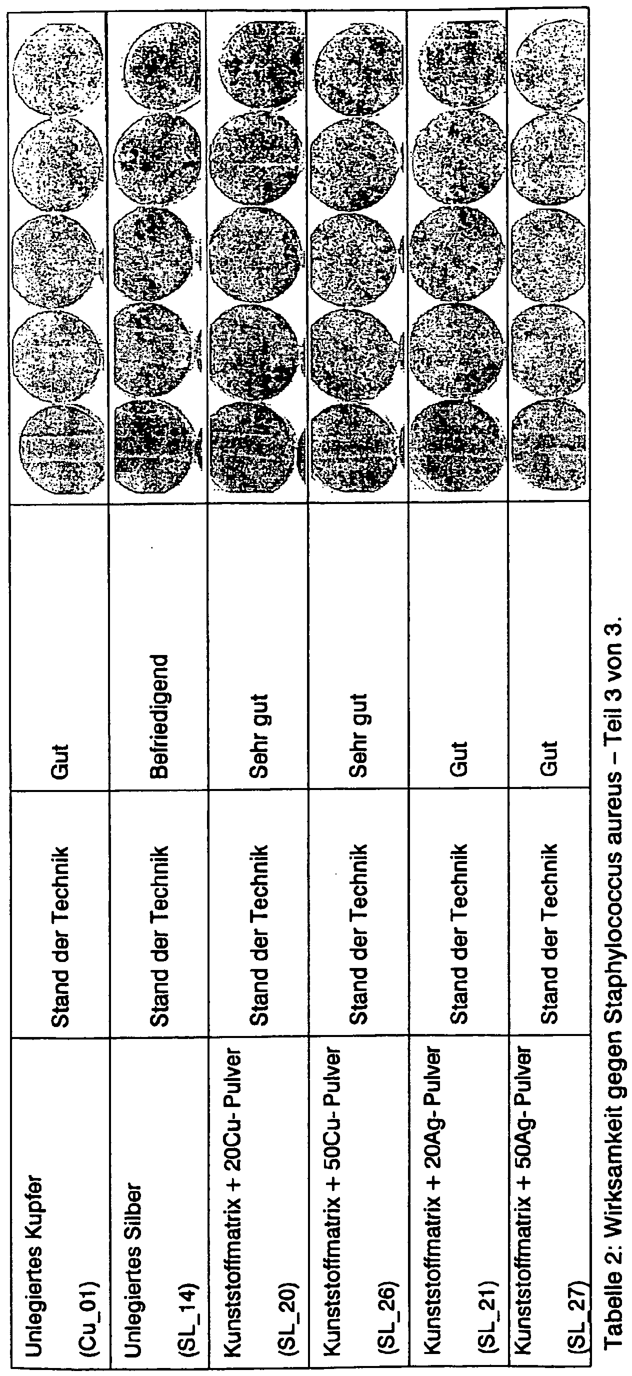

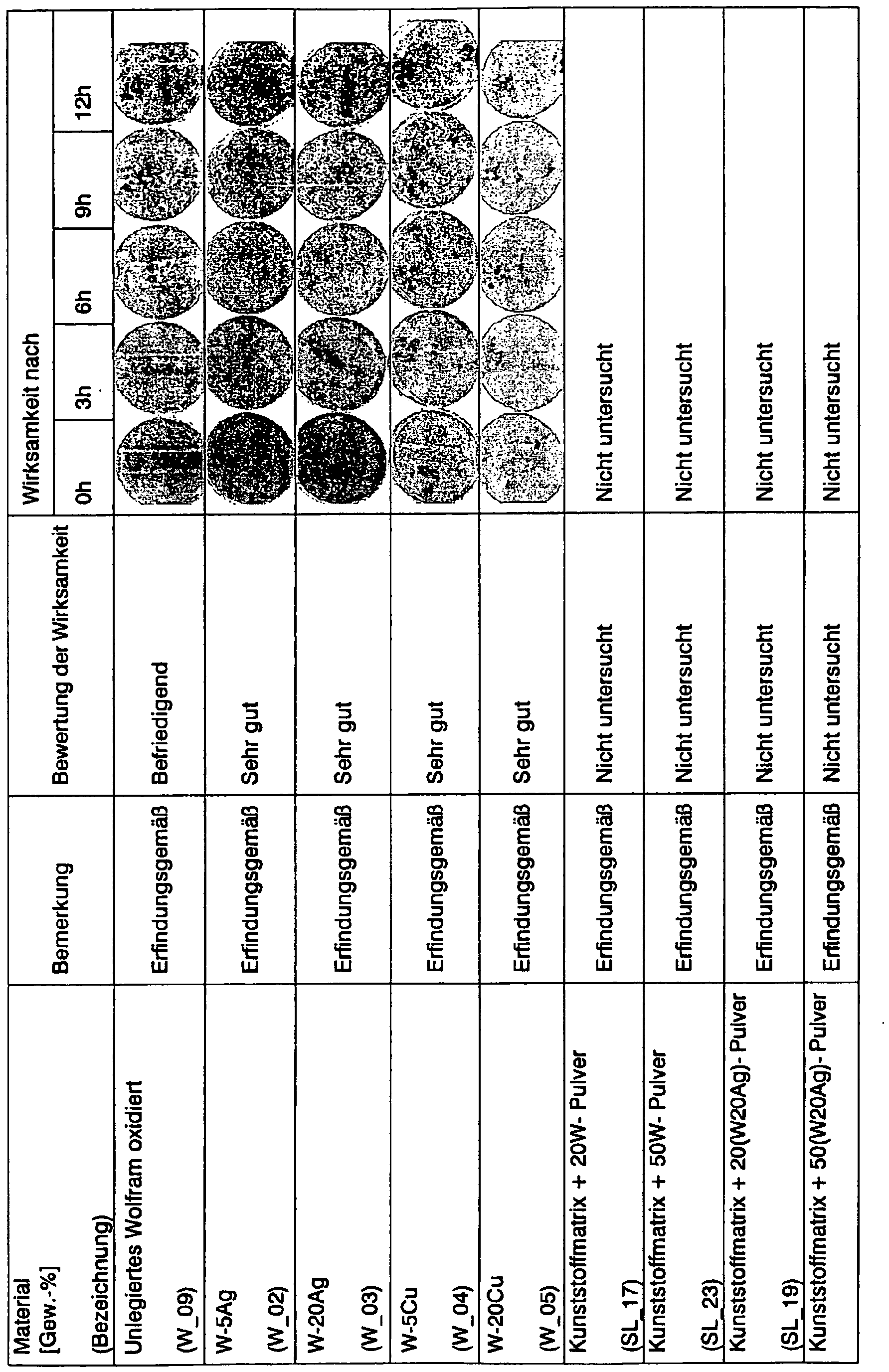

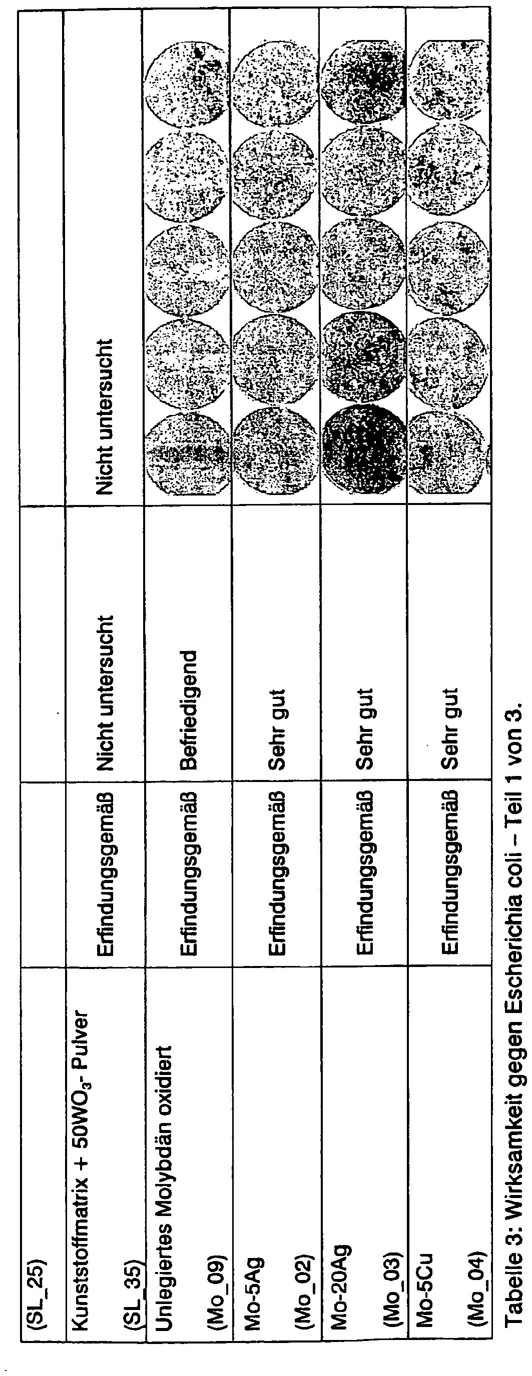

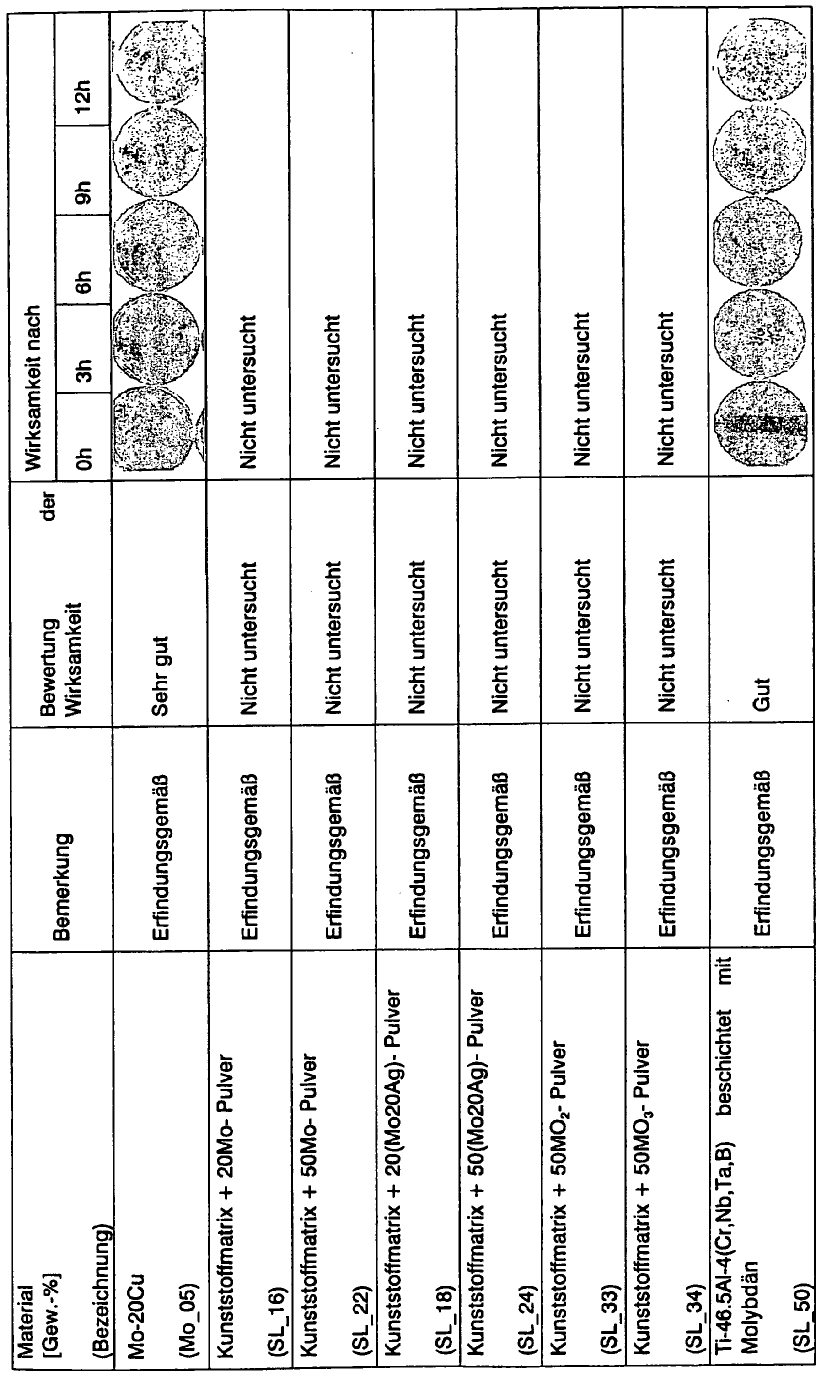

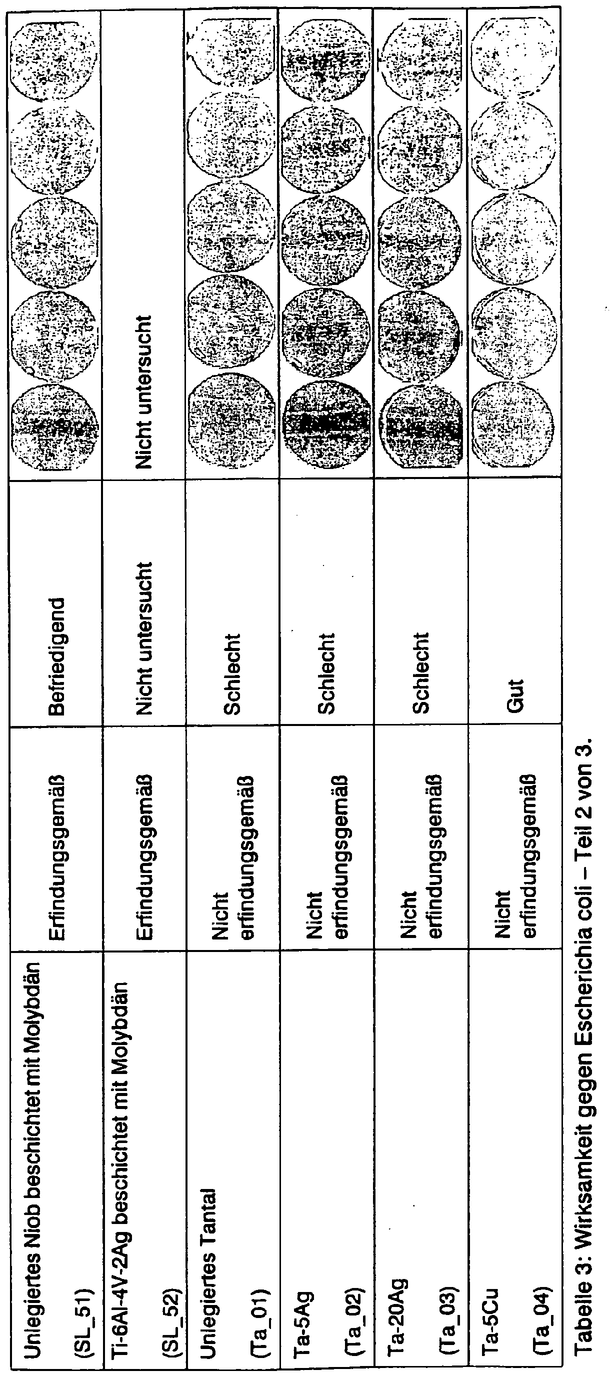

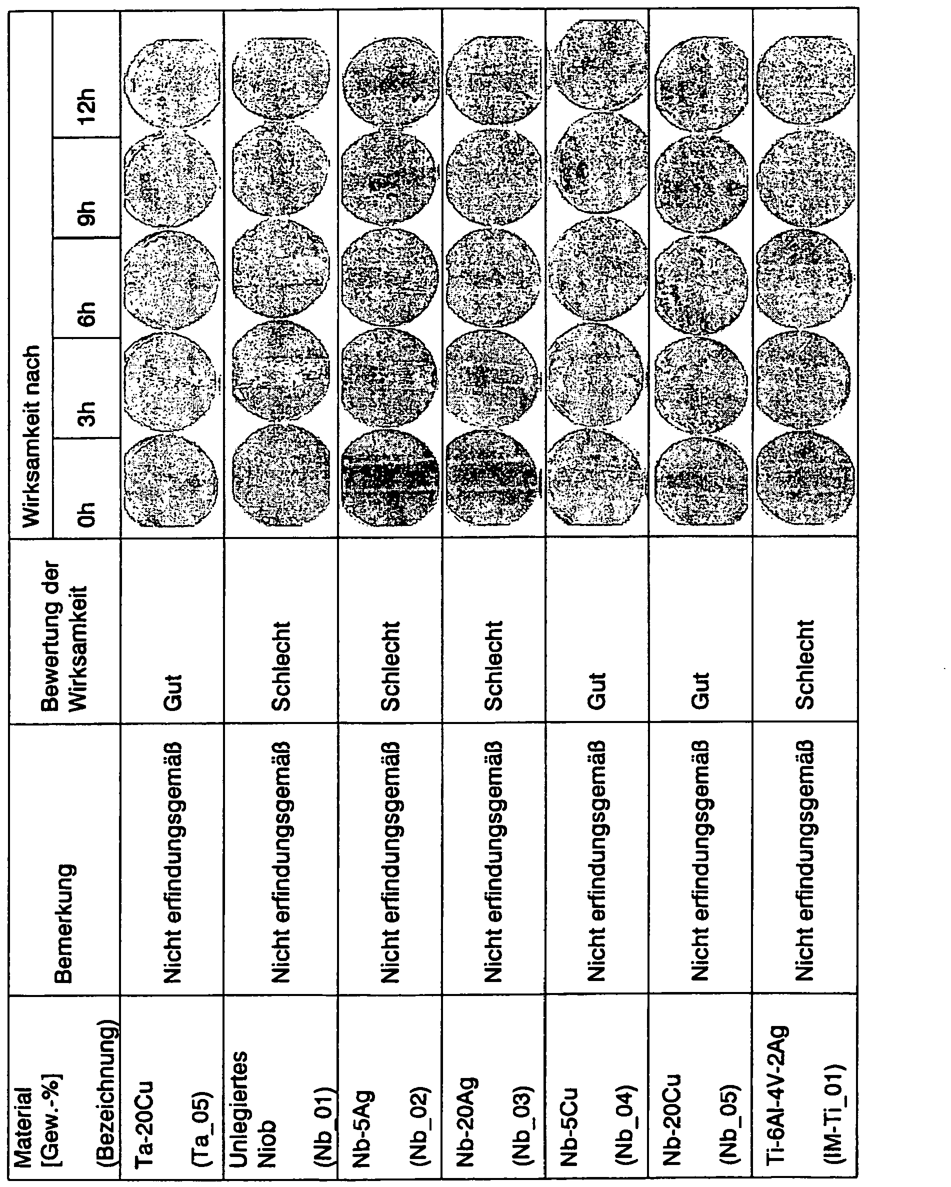

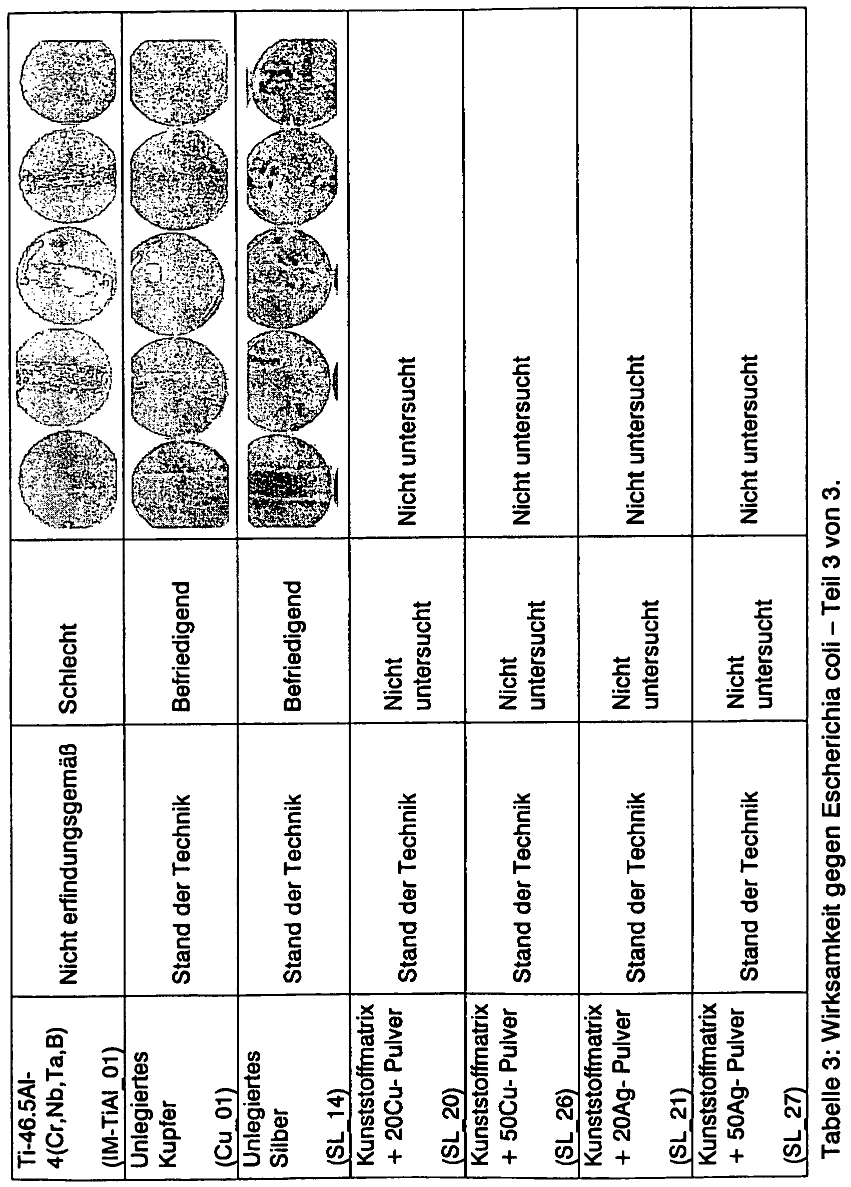

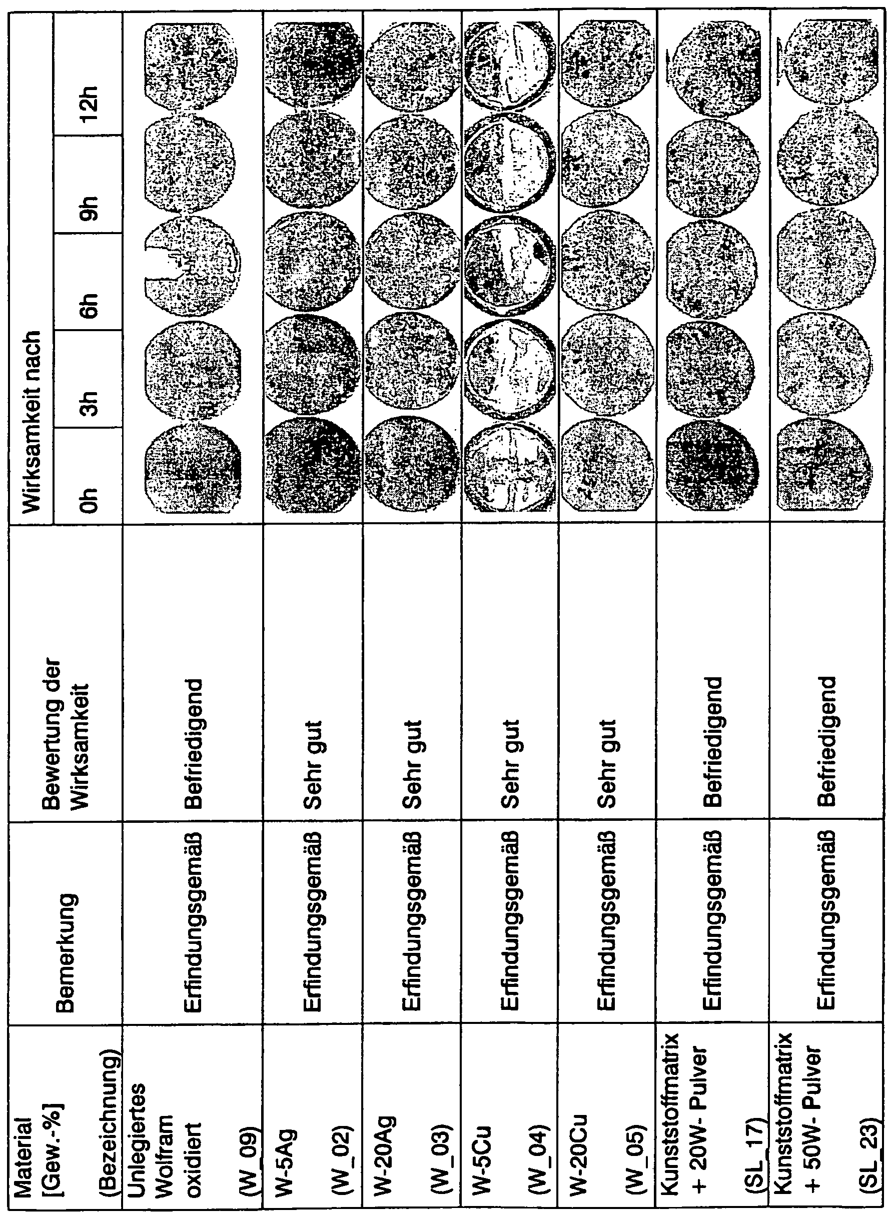

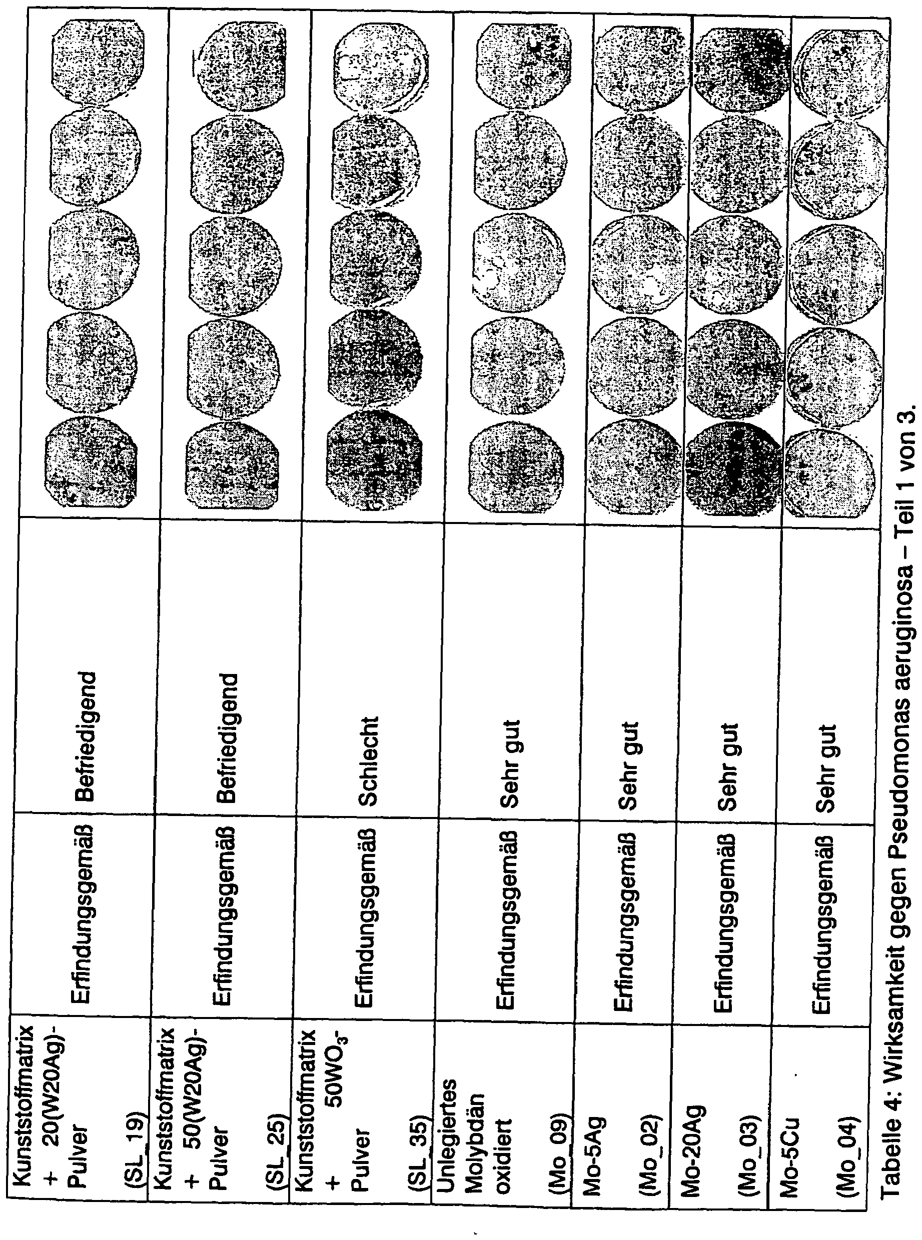

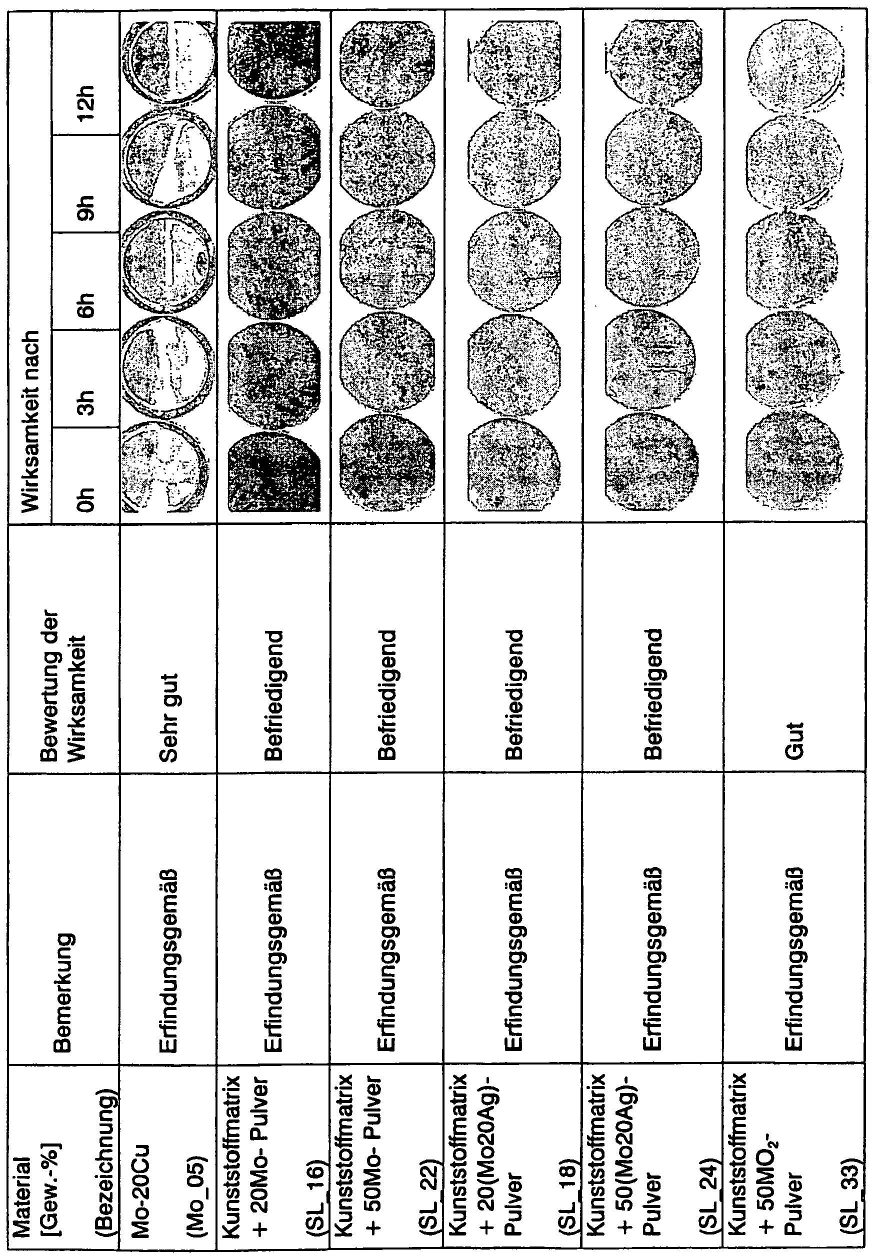

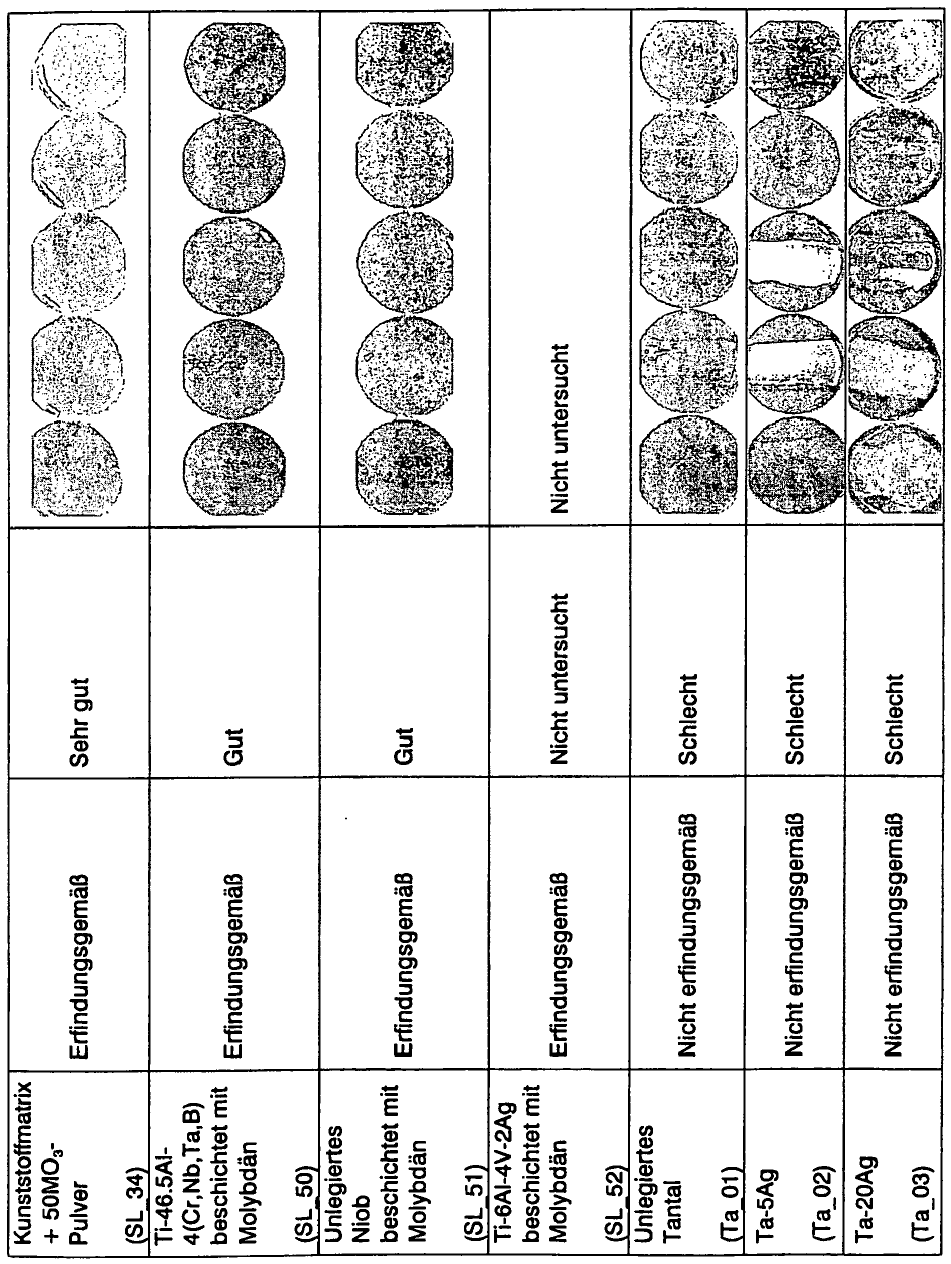

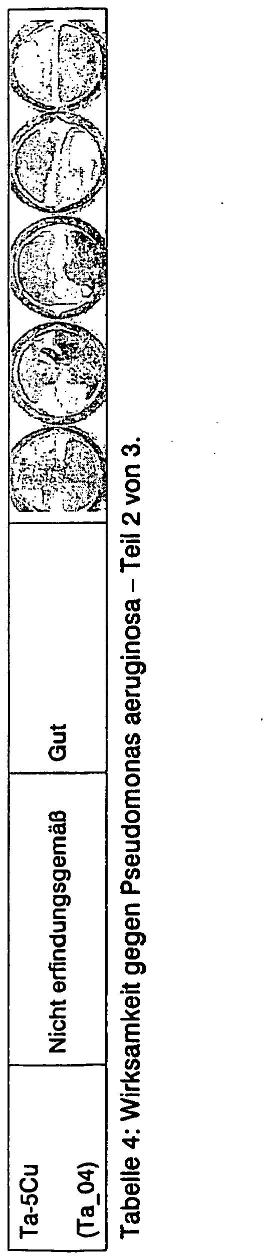

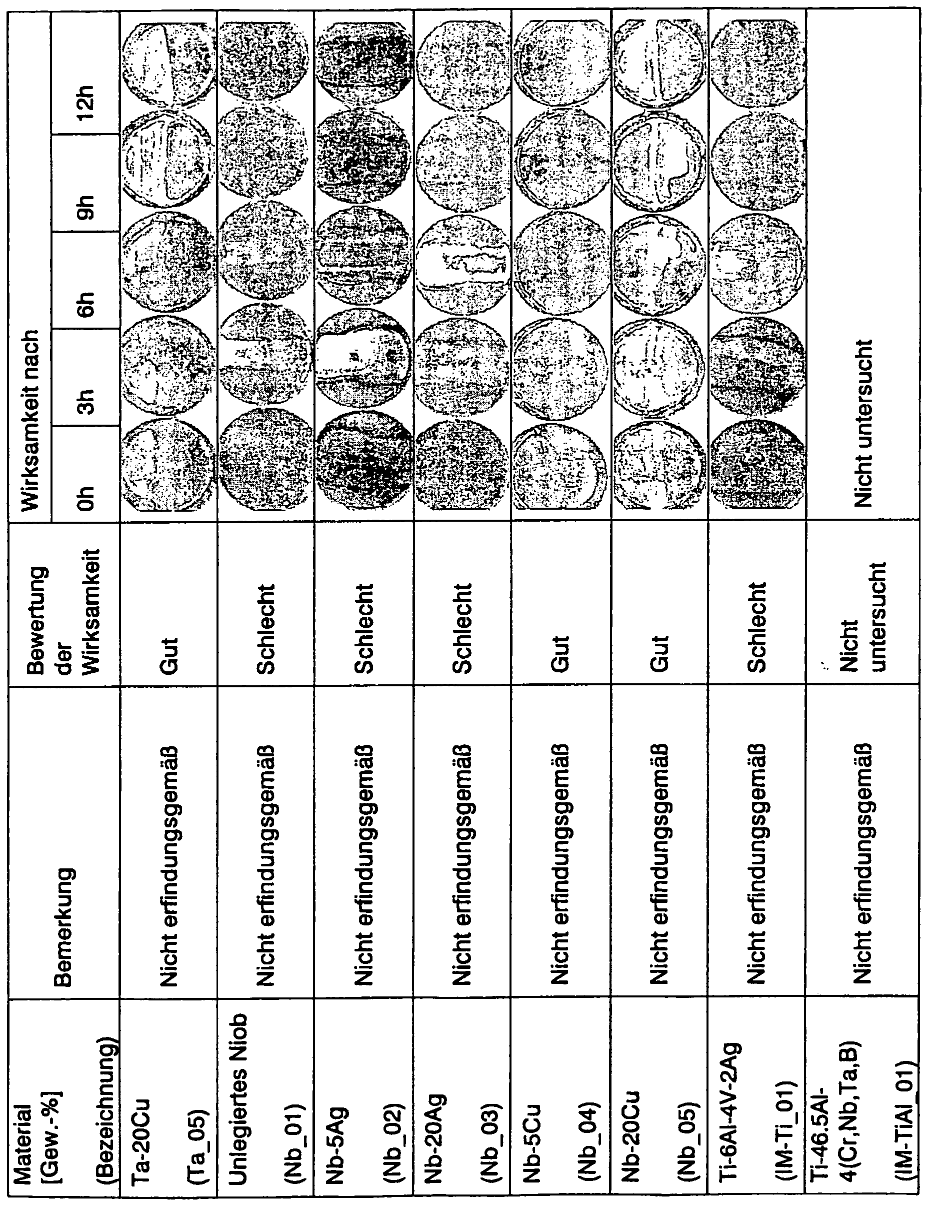

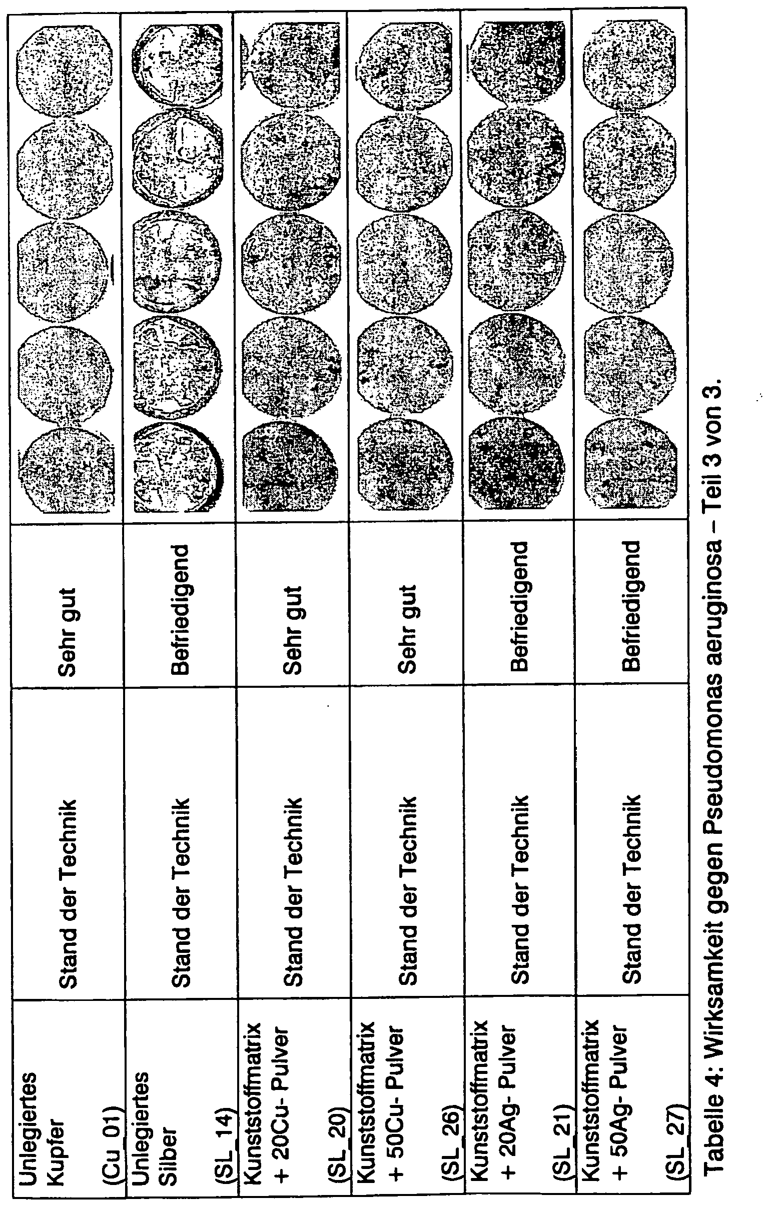

## Patentansprüche

1. Verwendung eines anorganischen Stoffes, der in Kontakt mit einem wässrigen Medium die Bildung von Wasserstoff-Kationen bewirkt, zur Erzielung einer antimikrobiellen Wirkung, **dadurch gekennzeichnet, dass** der Stoff Molybdän und/oder Wolfram enthält.

2. Verwendung nach Anspruch 1, **dadurch gekennzeichnet, dass** das wässrige Medium Wasser, eine Lösung, eine Suspension, eine Körperflüssigkeit oder flüssiges Gewebe ist oder dass das wässrige Medium in Form eines adsorbierten Feuchtigkeitsfilms auf der Stoffoberfläche vorliegt.

3. Verwendung nach einem der Ansprüche 1 oder 2, **dadurch gekennzeichnet, dass** durch die Bildung von Wasserstoff-Kationen der pH Wert des wässrigen Mediums < 6,0 beträgt oder dass die Löslichkeit des Stoffes im wässrigen Medium kleiner 0,1 Mol / Liter beträgt.

4. Verwendung nach einem der Ansprüche 1 bis 3, **dadurch gekennzeichnet, dass** die Oberfläche des Stoffes zumindest partiell oxidiert ist und/oder dass der Stoff Mo-Oxid, insbesondere MoO₂ und / oder MoO₃, oder W-Oxid, insbesondere Wolfram-Blauoxid und / oder WO₃, ist und/oder dass der Stoff Molybdän, eine Molybdänlegierung und / oder eine Molybdänverbindung ist, wobei die Oberfläche eine Mo-Oxidschicht aufweist, und/oder dass der Stoff Wolfram, eine Wolframlegierung und / oder eine Wolframverbindung ist, wobei die Oberfläche eine Wolfram-Oxidschicht aufweist.

5. Verwendung nach Anspruch 4, **dadurch gekennzeichnet, dass** die Molybdänlegierung Mo-0,1 bis 1 Gew-%La₂O₃, Mo-0,5 Gew.%Ti-0,08Gew.%Zr-0,01 bis 0,04Gew.%C, Mo-5 bis 50Gew.%Re oder Mol,2Gew.%Hf-0,02 bis 0,15Gew.%C ist und/oder dass die Molybdänverbindung Molybdänkarbid, Molybdännitrid, Molybdänsilizid und / oder Molybdänsulfid ist und/oder dass die Wolframlegierung W-0,1 bis 1 Gew.%La₂O₃ oder W-1 bis 26Gew.%Re ist und/oder dass die Wolframverbindung Wolframkarbid, Wolframnitrid, Wolframsilizid und / oder Wolframsulfid ist.

6. Verwendung nach einem der Ansprüche 1 bis 5, **dadurch gekennzeichnet, dass** eine weitere Phase vorliegt, die chemisch edler als der Stoff ist, wobei die Phase insbesondere die Phase Ag, Cu, Sn, und / oder eine Legierung dieser Metalle ist, und wobei der Stoff bevorzugt in Form eines Mo-Ag, Mo-Cu, Mo-Sn, W-Ag, W-Cu und / oder W-Sn Werkstoffs vorliegt, wobei der Mo- bzw. W-Gehalt vorzugsweise 10 bis 90 At.% beträgt.

7. Verwendung nach einem der Ansprüche 1 bis 6, **dadurch gekennzeichnet, dass** der Stoff in dichter Form und/oder in poröser Form und/oder als Schicht oder Bestandteil einer Schicht vorliegt.

8. Verwendung nach Anspruch 7, **dadurch gekennzeichnet, dass** die Schicht aus Mo-Oxid und / oder W-Oxid besteht, wobei die Schicht vorzugsweise aus Mo und Mo-Oxid oder W und W-Oxid besteht, und/oder wobei die Schicht vorzugsweise durch Elektronenstrahl-Verdampfung, Sputtern, chemische Dampfabscheidung, Elektrophorese, Slurry-Technik, Solgel-Technik oder Plasmaspritzen abgeschieden ist, und/oder wobei die Schicht vorzugsweise eine spongiöse, porige Struktur mit einer Porengröße von 50 bis 900 µm aufweist.

9. Verwendung nach Anspruch 7 oder 8, **dadurch gekennzeichnet, dass** der Stoff in der Schicht in Form inselartiger, im Wesentlichen nicht zusammenhängender Agglomerate vorliegt, wobei die mittlere Größe der einzelnen Stoffagglomerate vorzugsweise weniger als 5 µm beträgt und/oder wobei die Stoffagglomerate vorzugsweise durch Aufbringen eines Schlickers oder Abscheidung aus der Gasphase und optionaler anschließender Glühung ausgebildet sind.

10. Verwendung nach einem der Ansprüche 1 bis 9, **dadurch gekennzeichnet, dass** das Stoff im Verbund mit einem oder mehreren Werkstoffen als Verbundwerkstoff vorliegt, wobei der Massegehalt des Stoffes vorzugsweise 0,1 bis 50 Vol.% beträgt und/oder wobei der Verbundwerkstoff vorzugsweise eine Polymermatrix aufweist, wobei die Polymermatrix vorzugsweise ein hochvernetztes Polyethylen ist.

11. Verwendung nach Anspruch 10, **dadurch gekennzeichnet, dass** der Verbundwerkstoff Al₂O₃-MoO₃, Al₂O₃-WO₃, ZrO₂-MoO₃, ZrO₂-WO₃, Al₂O₃-Mo-MoO₃, Al₂O₃-W-WO₃, ZrO₂-Mo-MoO₃, ZrO₂-W-WO₃, TiO₂-MoO₃, TiO₂-WO₃, TiO₂-Mo-MoO₃, TiO₂-W-WO₃, SiO₂-MoO₃, SiO₂-WO₃, SiO₂-Mo-MoO₃ oder SiO₂-W-WO₃ ist, wobei der MoO₃ oder WO₃ Anteil vorzugsweise 0,001 bis 50 Mol.% beträgt und das Molverhältnis ZrO₂, Al₂O₃, TiO₂ oder SiO₂ zu MoO₃ oder WO₃ vorzugsweise 1 bis 100 beträgt.

12. Verwendung nach einem der Ansprüche 1 bis 11, **dadurch gekennzeichnet,**
- **dass** der Stoff als Zusatzstoff für Implantate verwendet wird, oder
- **dass** der Stoff als Zusatzstoff für einen saugfähigen Hygieneartikel oder eine Wundauflage verwendet wird, wobei diese(r) Polymerfasern oder -gitter enthält, auf deren Oberfläche der Stoff abgeschieden ist oder die den Stoff enthalten, oder
- **dass** der Stoff als Zusatzstoff für einen Lack, Beschichtungs- oder Klebstoff verwendet wird, wobei der Lack, Beschichtungs- oder Klebstoff vorzugsweise 0,01 bis 70 Vol.% des Stoffes enthält, oder
- **dass** der Stoff als Zusatzstoff für ein Körperpflegemittel verwendet wird, oder
- **dass** der Stoff als Zusatzstoff für einen Filter verwendet wird, oder
- **dass** der Stoff als Zusatzstoff für einen Bekleidungsartikel verwendet wird, wobei dieser Polymerfasern enthält, auf deren Oberfläche der Stoff abgeschieden ist oder die den Stoff enthalten, oder
- **dass** der Stoff als Zusatzstoff für Einrichtungsgegenstände verwendet wird, oder
- **dass** der Stoff als Zusatzstoff für ein Produkt verwendet wird, das im häufigen Berührungskontakt mit Lebewesen steht, oder
- **dass** der Stoff als Zusatzstoff in Wundsprays verwendet wird, oder
- **dass** der Stoff als Zusatzstoff in Behältnissen für Nasensprays verwendet wird, oder
- **dass** der Stoff als Zusatzstoff in Polyurethan enthaltenden Kabeln verwendet wird.

13. Verwendung nach Anspruch 12, **dadurch gekennzeichnet, dass** das Implantat ein Katheter, ein Stent, insbesondere ein Koronarstent aus Nitinol, der mit dem Stoff beschichtet ist, ein Knochenimplantat, ein Zahnimplantat, eine Gefäßprothese oder eine Endoprothese ist.

14. Verwendung nach Anspruch 13, **dadurch gekennzeichnet, dass** der Katheter ein Portkatheter ist, der einen Topf mit Silikonmembran und einen angeschlossenen Schlauch umfasst, wobei der Topf aus einem Polymerwerkstoff oder einem mit einem Polymerwerkstoff ummantelten Werkstoff, der den Stoff enthält, besteht und / oder zumindest ein Teil mit einer Schicht versehen ist, die den Stoff enthält und / oder der Polymerwerkstoff und / oder die Silikonmembran den Stoff enthält.

15. Verwendung nach Anspruch 12, **dadurch gekennzeichnet, dass** das Produkt ein Schalter, eine Armatur, eine Kreditkarte, eine Tastatur, ein Handygehäuse, eine Münze, ein Geldschein, eine Türschnalle oder ein Teil der Innenausstattung eines öffentlichen Verkehrsmittels ist.
